# EUROPEAN PATENT APPLICATION

(11) **EP 3 415 617 A1**
(43) Date of publication of application: **19.12.2018**
(21) Application number: 17176417.8
(22) Date of filing: 16.06.2017
(51) Int. Cl.: C12N 5/0783

(54) **A METHOD AND A DEVICE FOR INCREASING EX VIVO EXPANSION OF T CELLS BY USING ADHESIVE NANOSTRUCTURED SURFACES AND COSTIMULATORY SIGNALS**

(71) Applicant: Max-Planck-Gesellschaft zur Förderung der Wissenschaften e.V., 80539 München (DE)
(72) Inventor: SPATZ, Joachim P., 70569 Stuttgart (DE); GUASCH, Judith, 43711 Banyeres del Penedès (ES)
(74) Representative: Vossius & Partner Patentanwälte Rechtsanwälte mbB

(57) **Abstract**

The present invention relates to a method for expansion of T cells. The method comprises the steps of: (i) culturing a T cell in the presence of a phorbol ester, a divalent ionophore, and optionally one or more protein transport inhibitors; and (ii) culturing the T cell in the presence of an agent capable of activating CD3 and/or an agent capable of activating CD28. In another aspect, the present invention relates to a device, preferably a device having a nanostructured surface that comprises or consists of an array of nanoparticles (NPs), wherein an Arg-Gly-Asp (RGD) peptide is attached on the interparticle area of the surface of the device and CD3 and/or CD28 activating agents are immobilized on the surface of the NPs. The present invention also relates to the use of said device for T cell expansion. Finally, also T cells obtainable or obtained with a method for T cell expansion according to the present invention and/or compositions comprising said T cells as well as medical uses thereof are provided.

## Description

The present invention relates to a method and a device for *ex vivo* expansion of primary human T cells, such as CD4+ T cells. Further, the present invention also relates to T cells and compositions comprising T cells obtainable by said method. In particular, the present invention relates to a method fpr T cell expansion that comprises the steps of: (i) culturing a T cell in the presence of a phorbol ester, a divalent ionophore, and optionally one or more protein transport inhibitors; and (ii) culturing the T cell in the presence of an agent capable of activating CD3 and/or an agent capable of activating CD28. In another aspect, the present invention relates to a device, preferably a device having a nanostructured surface that comprises or consists of an array of nanoparticles (NPs), wherein an Arg-Gly-Asp (RGD) peptide is attached on the interparticle area of the surface of the device and CD3 and/or CD28 activating agents are immobilized on the surface of the NPs. The present invention also relates to the use of said device for T cell expansion. Finally, also T cells obtainable or obtained with a method for T cell expansion according to the present invention and/or compositions comprising said T cells as well as medical uses thereof are provided.

Adoptive T cell therapy represents a promising treatment for cancer, chronic viral infections, and even autoimmune diseases, which should be improved in several aspects in order to be used as a standard procedure in the clinics (see References [1]). One of these challenges in adoptive T cell therapy is to obtain a fast and affordable expansion of primary human T cells *in vitro* (see References [2]).

An effective T cell activation and proliferation requires T cell receptor (TCR) ligation (see Reference [3]) and costimulatory signals, which include membrane receptors of the antigen presenting cells (APCs) that bind to the T cell surface (see References [4]) and the presence of cytokines from inflammatory, homeostatic, or autocrine sources (see Reference [5]).

The present state-of-the-art method used for *in vitro* activation and proliferation of T cells, specially for polyclonal expansions, involves culturing T cells in the presence of beads coated with stimulating agents such as the anti-CD3 and anti-CD28 monoclonal antibodies (see References [1]) used in the commercial Dynabeads (Thermo Fisher Scientific, US, Article Nr. 11131D (in January 2017)). However, since *ex vivo* expansion of T cells is a cost and time intensive step, specially for populations with a low incidence, that needs to be performed during adoptive T cell therapy, there is a high need to provide new means and methods for further improving the *in vitro* activation and proliferation of T cells in order to make the treatment accessible to the clinics.

Recently, new methods for *ex vivo* stimulation and proliferation of T cells that employ functionalized nanostructured surfaces (also referred to as nanoarrays) produced by block copolymer micellar (nano-) lithography (BCML) have been described (see References [6]).

In Reference [6]a), Delcassian et al. described a nanostructured surface for the stimulation of T cells that was produced by BCML and functionalized with F(ab')₂ fragments derived from the UCHT-1 antibody that binds the CD3ε component of the TCR complex and the integrin ligand ICAM-1.

In Reference [6]b), Deeg et al. disclosed a nanopatterned antigen array produced by BCML that was functionalized with major histocompatibility class II proteins (pMHC) and the use thereof for T cell activation.

Finally, in Reference [6]c), Matic et al. have reported anti-CD3 nanoarrays fabricated by BCML in which an anti-CD3 antibody was attached and the use thereof for stimulating CD4+ T cells, as e.g. indicated by IL-2 secretion. Matic et al. observed that IL-2 secretion by the CD4+ T cells cultured on the anti-CD3 nanoarray could be increased if soluble anti-CD28 antibody was added during culturing.

While these nanostructured surfaces would allow for a more precise control at the nanoscale (e.g. definition of the distance between two stimulatory signals) than the commercially available beads, the T cell activation and proliferation rates achieved by these surfaces are around 30 times lower than the rates achieved by the present state-of-the-art Dynabeads method. This is illustrated by the IL-2 values reported by Matic et al. and the results presented herein for the method using Dynabeads, which were mixed with CD4+ T cells following the supplier's recommendations in a static culture system without external IL-2. Thus, there is a high need to provide new nanostructured surfaces (e.g. produced by BCML) and methods using the same that allow for increased and/or more cost-efficient *ex vivo* activation and expansion of T cells such as CD4+ T cells.

Accordingly, the technical problem underlying the present invention is the provision of improved means and methods for *ex vivo* expansion of T cells (e.g. CD4+ T cells). Moreover, the present invention has the objective to provide means and methods for improving the *ex vivo* expansion of T cells (e.g. CD4+ T cells) using a functionalized nanostructured surface such as a functionalized nanostructured surface of a device (e.g. obtainable by BCLM).

The technical problem is solved by provision of the embodiments characterized in the claims.

According to a first aspect, the present invention relates to a method for the expansion of T cells, the method comprising the steps:
(i) culturing a T cell in the presence of a phorbol ester and a divalent ionophore; and
(ii) culturing the T cell in the presence of an agent capable of activating CD3 and/or an agent capable of activating CD28.

Optionally, in step (i) also one or more protein transport inhibitors may be present during culturing.

It is demonstrated by the disclosure of the present application that T cells (e.g. CD4+ T cells) can be efficiently expanded *in vitro* using this method. Importantly, as demonstrated in the appended Examples and Figures, the method of the present invention is even superior to the present state-of-the-art methods for T cell expansion involving commercially available Dynabeads that are functionalized with anti-CD3 and anti-CD28 antibodies (Thermo Fisher Scientific, US, Article Nr. 11131D (in January 2017)). Accordingly, the present invention may contribute in advancing T cell therapies by improving the step of *in vitro* expansion of T cells (e.g. CD4+ T cells).

The method for T cell expansion of the present invention as defined above differs from the state-of-the-art Dynabeads method at least in that it additionally involves culturing a T cell in the presence of a phorbol ester (e.g. phorbol-12-myristate-13-acetate (PMA)), a divalent ionophore (e.g. ionomycin), and optionally one or more protein transport inhibitors (e.g. brefeldin A and/or monensin). As shown by the appended Examples and Figures, the present inventors have surprisingly found that by combining an anti-CD3/anti-CD28-based approach with a phorbol ester (e.g. PMA)/divalent ionophore (e.g. ionomycin)-based method, the efficiency of the *ex vivo* expansion of T cells (e.g. CD4+ T cells) and/or the level of stimulation/activation of T cells (e.g. CD4+ T cells) can be synergistically improved. This improvement could be achieved with different expansion systems such as the state-of-the-art Dynabeads method, or methods using a nanostructured surface. Even though the T cell activation potential of some phorbol esters and ionomycin has been known (see References [7]a) to e)), this finding is particularly surprising, because the treatment of T cells with CD3/CD28 activating agents on one side and the stimulation of T cells in the presence of a phorbol ester and a divalent ionophore on the other side have been considered to be alternatives for T cell activation. In other words, activation and expansion of T cells in the presence of a phorbol ester and a divalent ionophore has been known as a "bypass" to stimulation cell membrane receptors of T cells with CD3/CD28 activating agents (see Reference [8]). Accordingly, in view of the prior art, a skilled person would have had no motivation to consider combining both stimulation methods to improve the expansion of T cells. The increased T cell expansion achieved by the method of the present invention can speed up T cell expansion, which is highly desirable in a clinical setting of T cell therapies.

In preferred embodiments of the method of the present invention, the CD3 activating agent (e.g. an anti-CD3 antibody) and/or the CD28 activating agent (e.g. an anti-CD28 antibody) are attached to a surface, preferably to nanoparticles (NPs), such as gold NPs (AuNPs). The NPs are preferably part of a nanostructured surface (e.g. generated by BCLM). The methods for T cell activation and expansion of the present invention using a nanostructured surface achieved a by far superior T cell activation and expansion (as determined by the levels of secreted IL-2) compared to previously described methods for T cell activation and/or expansion involving nanoarrays (i.e. devices/members having a nanostructured surface) such as, e.g. the method described by Matic et al. (see Reference [6]c)). Specifically, the present invention demonstrates that a method involving (i) culturing T cells in the presence of a phorbol ester (e.g. PMA) and a divalent ionophore (e.g. ionomycin), and (ii) the subsequent culturing of these T cells in a culture medium comprising soluble anti-CD28 antibody and in the presence of a nanostructured surface comprising NPs (e.g. AuNPs) covered by an anti-CD3 antibody resulted in an IL-2 secretion that is about 30-times higher than in Matic et al (see appended Examples and Figures). Even more importantly, the embodiments of the present invention using a CD3 activating agent (e.g. an anti-CD3 antibody) and/or a CD28 activating agent (e.g. an anti-CD28 antibody) being attached to a nanostructured surface in combination with a treatment (e.g. pretreatment) with phorbol ester (e.g. PMA) and a divalent ionophore (e.g. ionomycin) are not only superior to previously described methods using nanostructured surfaces but even achieve T cell stimulation and expansion that is superior to the state-of-the-art Dynabeads method. Even in the presence of only a CD3 activating agent or a CD28 activating agent attached to the nanostructured surface (e.g. the nanostructured surface of a device according to the present invention), the IL-2 secretion levels at least compare to the state-of-the-art Dynabeads method using both anti-CD3 and anti-CD28 antibodies for T cell stimulation. In the embodiments employing a CD3 activating agent and a CD28 activating agent, wherein at least the first is attached to a surface (e.g. a nanostructured surface of the present invention), a T cell activation and expansion superior to the state-of-the art Dynabeads method was surprisingly achieved. Accordingly, the present invention provides for the first time methods for *ex vivo* T cell expansion using functionalized nanostructured surfaces (i.e. nanostructured surfaces functionalized with activating agents, such as antibodies) that achieve comparable and actually even superior T cell expansion efficiencies than the state-of-the art Dynabeads method. Since the method of the present invention is particularly efficient when used with a device having a nanostructured surface (e.g. a device with a nanostructured surface as described herein), it allows for the first time to combine the advantages of nanostructured surfaces with a highly efficient T cell amplification/expansion *in vitro.* Using nanostructured surfaces, for example, provides the advantage of a tight control on the nanoscale (e.g. a tight control of the number, the distance between and/or the orientation of activating agents being attached to NPs). Moreover, in contrast to the state-of-the-art Dynabeads method, no step of separating the beads from the cells before clinical application is required. Therefore, the potential risk of an incomplete removal of beads from expanded T cells before injection can be prevented. Thus, the embodiments using nanostructured surfaces of the present invention even provide additional advantages compared to the state-of-the-art Dynabeads method, which itself can however already be improved by being combined with the method of the present invention.

In a second aspect, the present invention provides a device (preferably a device for expansion of T cells), wherein a peptide comprising or consisting of arginine-glycine-aspartic acid (the peptide comprising or consisting of arginine-glycine-aspartic acid is also referred to as Arg-Gly-Asp or RGD in the context of the present invention) is attached to the (upper) surface of the device, preferably a surface comprising or consisting of TiO₂. It is particularly envisaged in the context of the present invention that the RGD employed comprises a phosphonic acid, because this allows for a highly stable attachment (also under aqueous conditions such as during cell culture) of such RGD on a surface comprising and/or consisting of TiO₂. In a preferred embodiment, said upper surface of the device is a nanostructured surface comprising quasi-hexagonally ordered NPs. These NPs (preferably AuNPs) may preferably be functionalized with one or more agents that stimulate T cell (e.g. CD4+ T cell) expansion. The RGD peptides are preferably (covalently) attached/linked to the interparticle space, i.e. the surface between the NPs. The one or more agents that stimulate the expansion of T cells used in context of the device of the present invention are preferably an agent capable of activating CD3 and/or an agent capable of activating CD28. Preferably, the devices and/or nanostructured surfaces of the present invention are produced by BCML.

The technical contribution of the device of the present invention is illustrated by the appended Examples and Figures. The present inventors could demonstrate that when a device of the present invention having a nanostructured surface according to the present invention is combined with a method for T cell expansion of the present invention, a highly efficient T cell activation and expansion that is superior to previously described methods is obtained. Accordingly, it has surprisingly been found that the device of the present invention has an advantageous technical contribution on T cell expansion over prior art devices. It is believed that RGD (preferably cyclic RGD) has a technical contribution to T cell activation over previously used non-adhesive surfaces (e.g. polyethylenglycol coated surfaces as used in Matic et al. (see Reference [6]c)). Specifically, it is believed to increase the strength of the immune synapse (IS) (or a mimicking signal such as the one obtained using a CD3 activating agent in the context of the method of the present invention) by increasing the duration of the interaction between a T cell and the (upper) surface (preferably nanostructured surface) of a device being covered/functionalized with T cell stimulatory agents (e.g. a CD3 activating agent). It is believed that the device according to the present invention is superior to previously described devices, such as the nanoarrays as described in References [6], with respect to its capability to activate and expand T cells.

As discussed above, and illustrated in the appended Examples and Figures, the device of the present invention has advantageous technical contributions in T cell (e.g. CD4+ T cell) activation and expansion. Accordingly, the present invention also relates to the use of such a device for T cell (e.g. CD4+ T cell) expansion. Similarly, the present invention also relates to the use of the device for T cell (e.g. CD4+ T cells) activation. Accordingly the present invention also relates to the use of the device for T cell (e.g. CD4+ T cells) activation and/or expansion.

As mentioned above, according to a first aspect the present invention provides a method for the expansion/amplification of T cells. Said method comprises:
(i) culturing a T cell in the presence of a phorbol ester and a divalent ionophore; and
(ii) culturing the T cell in the presence of an agent capable of activating CD3 and/or an agent capable of activating CD28.

It is particularly envisaged that the method may be a method for *ex vivo*/*in vitro* expansion of T cells, i.e. the expansion of T cells in cell culture systems. "Expansion" of T cells means the proliferation/amplification of T cells (and also includes the stimulation and activation of T cells that is interrelated with T cell expansion). Accordingly, a method for T cell expansion as described herein may also be referred to as a method for (*ex vivo*) proliferation/amplification of T cells. The expansion as referred to in the present invention is preferably a polyclonal expansion, i.e. that T cells with different T cell receptor specificities and/or T cells of different T cell subgroups are expanded/proliferated. Another alternative expression that may be used instead of the term "expansion" is the term "generation", because an increase of T cells in a culture system means that new T cells are produced. The terms expansion, proliferation, amplification, and generation all express that the number of cells increases during culturing.

Methods to determine and measure T cell expansion, i.e. an increase in the number of T cells, are well known in the art. For instance expansion may be determined by measuring cell numbers before and at one or more time points during or after culturing in step (ii) of the method of the present invention. Moreover, cell expansion/proliferation rates and/or a cell division index may be determined and compared for stained cells (e.g. with carboxyfluorescein succinimidyl ester (CFSE)) before and during being subjected to the present invention (e.g. before and (about) 7 days of culturing in step (ii)). Alternatively, the cells cultured according to the present invention may also be compared to respective control cells. Such control cells can e.g. be the same T cells as subjected to the present invention but without being activated, i.e. without being subjected to the method of the present invention (negative controls). Control cells can also be cells (e.g. the T cells subjected into the method of the present invention) cultured by state-of-the-art methods for T cell expansion like the herein mentioned Dynabeads method (positive controls). In this event comparing the cell proliferation rate and/or the cell division rate may be a measure to demonstrate the improved expansion achieved by the present invention. Illustrative examples for methods to determine T cell expansion/proliferation rates and/or cell division indexes are provided in the appended Examples and Figures. In brief, such a method may involve staining T cells with a fluorescent dye that is suited for following T cell proliferation/expansion, e.g. CFSE, prior to seeding and culturing according to the present invention. CFSE is a fluorescent cell staining dye that is cell permeable and covalently couples, via its succinimidyl group, to intracellular molecules via intracellular lysine residues and other amine sources. Due to this covalent coupling reaction fluorescent CFSE can be retained within cells for extremely long periods. It is well known in the art that CFSE can be used for measuring T cell proliferation, e.g. by flow cytometry (see Reference [9]) and corresponding kits are commercially available (e.g. "CellTrace CFSE Cell Proliferation Kit, for flow cytometry, ThermoFisher Scientific (US), Article Nr. C34554 (in May 2017)). Cells stained with a fluorescent dye such as CFSE can be subsequently analyzed by flow cytometry to measure cell expansion and to determine cell expansion rates and/or a cell division index. To accurately measure a cell expansion rate or a cell division index preferably the number of cells that is seeded/subjected to the method of the present invention may be in a range that prevents cell saturation. The number of cells needs to be adapted to the size of the well and the amount of stimulatory agents provided. An illustrative example is provided in the appended Examples. For the proliferation analysis (i.e. for measuring cell expansion and/or to determine cell expansion rates and/or a cell division index) the labeled cells may be cultivated at least 1, preferably at least 2, more preferably at least 3, even more preferably at least 5, even more preferably at least 14, and most preferably at least 7 days after seeding. Further, for the proliferation analysis the labeled cells may be cultivated at most 1, preferably at most 2, more preferably at most 3, even more preferably at most 5, even more preferably at most 14, and most preferably at most 7 days after seeding. In a preferred embodiment labeled cells are incubated for 7 days for proliferation analysis. The number of cells, the expansion rate, and/or the cell division index may be determined by using software well-known in the art, such as FlowJo software (Version 9.8.5, FlowJo, LCC).

T cell expansion is tightly linked to T cell activation in that T cells that expand are also activated. In other words, T cell activation triggers T cell expansion. Therefore, to detect T cell expansion one may also take assays to detect T cell activation (see below) into account.

Activation of T cells means or involves the stimulation of signalling pathways linked to the recognition of a T cell receptor ligand, T cell receptor signalling and/or costimulatory signalling. T cell activation is tightly linked but not necessarily linked to T cell expansion. In other words, T cells can be activated without expansion. Whether T cells are only activated or also expanded may depend on the strength of the activation and in particular on the IL-2 levels produced. For instance, T cells expand if the IL-2 level is 0.5 ng/ml or higher 16 to 20 hours after seeding in cultures of cells that were seeded at 1 million cells/ml. Under these conditions lower IL-2 levels may indicate that T cells are only activated. The level of activation can be influenced by the specificity of the activation agents(s) (e.g. CD3 activating agent and/or CD28 activating agent), the concentration and orientation of activation agent(s) (e.g. CD3 activating agent and/or CD28 activating agent) provided, and/or the time of incubation in presence of activation agent(s) (e.g. CD3 activating agent and/or CD28 activating agent). Measures to detect T cell activation include the production or increased production of cytokines and new cell surface proteins including growth factor receptors, which are well known in the art (see Reference [8]) and may be selected depending on the type of T cells employed. For example, a measure for T cell activation may be selected from measuring levels of secreted IL-2 (wherein an increase in the level indicates T cell activation) and/or measuring levels of secreted INF-γ (wherein an increase in the level indicates T cell activation). Secreted IL-2 levels may, for example, be measured through standard techniques such as an enzyme linked immunosorbent assay (ELISA), using commercially available kits (e.g. Human IL-2 Quantikine ELISA Kit, R&D Systems, US). Standard techniques such as ELISA techniques may also be employed to detect the other stimulation/activation measures mentioned above, such as measuring secreted INF-γ levels. To detect an activation by production or increased production one or more of the measures for T cell activation (e.g. as mentioned above) may be compared with the respective levels of the cells before being subjected to the method of the present invention or with control cells as mentioned in the context of determining T cell expansion, above. To determine that cells are only activated but do not expand, one of the assays to identify T cell activation described above may be employed and in parallel T cell expansion may be analyzed. If no expansion (i.e. an increase in cell numbers) but activation is observed, T cells are only activated. As indicated above, a measure to differentiate between only activation and expansion of T cells is also level of secreted IL-2. As indicated above, T cells may expand if the IL-2 level is 0.5 ng/ml or higher in cultures of cells 16 to 20 hours after seeding that were seeded at 1 million cells/ml.
The method for T cell expansion (sometimes due to the activation being a requirement for expansion also referred to as method for activation and expansion herein) as described herein may also be employed to activate T cells. Even though T cell activation and expansion is tightly linked, T cells can in principle also be activated without expansion. Thus the present invention also relates to a method for the activation of T cells that comprises:
(i) culturing a T cell in the presence of a phorbol ester and a divalent ionophore; and
(ii) culturing the T cell in the presence of an agent capable of activating CD3 and/or an agent capable of activating CD28.

What is said herein for the method of expansion of T cells (especially any preferred embodiments) applies *mutatis mutandis* to the above-mentioned method for activation of T cells.

Moreover, the present invention also relates to a method for the expansion and/or activation of T cells that comprises:
(i) culturing a T cell in the presence of a phorbol ester and a divalent ionophore; and
(ii) culturing the T cell in the presence of an agent capable of activating CD3 and/or an agent capable of activating CD28.

What is said herein for the method of expansion of T cells (especially any preferred embodiments) and the method of activating T cells (especially any preferred embodiments) applies *mutatis mutandis* to the above-mentioned method for expansion and/or activation of T cells.

According to the present invention steps (i) and (ii) of the method for the expansion of T cells of the present invention may in principle be performed in any order, i.e. step (i) may be performed before step (ii), step (i) may be performed after step (ii), or steps (i) and (ii) may be performed simultaneously. Preferred is the performance of step (i) before, even more preferably directly before step (ii). In other words step (ii) is preferably performed subsequent to or after step (i). Accordingly, step (i) may also be formulated as prestimulating a T cell by culturing said T cell in the presence of a phorbol ester, a divalent ionophore, and optionally one or more protein transport inhibitors.

In the context of the present invention, the divalent ionophore to be employed may be selected from different divalent ionophores known in the art, preferably from any of the divalent ionophores previously described in the context of T cell activation and/or expansion as an alternative to CD3 activation. In the context of the present invention an ionophore means a lipid-soluble entity that transport ions across a cell membrane. An ionophore may also be referred to as "ion carrier" as they facilitate ion transport across hydrophobic cell membranes. The ionophore employed herein may be an ionophore that is known to be synthesized and/or synthesized by microorganisms. Alternatively, the ionophore herein may also be a synthetic ion carrier. In the context of the present invention the term divalent ionophore means an ionophore that facilitates the transport of divalent ions across a cell membrane (e.g. a T cell membrane). A preferred divalent ionophore employed in the context of the present invention is a divalent cation ionophore, i.e. an ionophore that transports divalent cations across the cell membrane. Even more preferably, a divalent ionophore employed in the context of the present invention is an ionophore that is capable of transporting Ca²⁺ ions across the cell membrane, preferably from the outside to the cytoplasm of cells, such as T cells. Accordingly, the divalent ionophore may preferably be a divalent ionophore capable of transporting Ca²⁺-ions to the cytoplasm of cells, i.e. may be a calcium (Ca²⁺) ionophore. A calcium ionophore or a Ca²⁺ ionophore in other words means an ionophore suited to increase the intracellular Ca²⁺ concentration. Even more preferably, divalent ionophores that are employed are ionomycin, A23187, or a combination thereof. Most preferably ionomycin is employed. Ionomycin and A23187 are members of the groups of divalent ionophores, divalent cation ionophores and Ca²⁺ ionophores. Ionomycin is a well-known commercially available ionophore that is, e.g. naturally produced by the bacterium *Streptomyces conglobatus.* Ionomycin is well known as a research tool to raise the intracellular level of calcium and can, for example, be obtained as a free acid, or as a Ca²⁺ salt. Both forms can be employed in the context of the present invention. A23187 is also a commercially available ionophore with selectivity for divalent cations. A23187 is also known as Calcimycin, calcium ionophore, and calcium ionophore A23187 in the art. It is, for example, commercially available as free acid, Ca²⁺ salt, and a 4-brominated analog. All these forms of A23187 may be employed in the context of the present invention.

A skilled person is aware of the typical concentration of divalent ionophore that needs to be selected in order to activate T cells and to induce T cell expansion. In particular, a skilled person is also aware that depending on the respective divalent ionophore used, the concentration may need to be adapted. Examples for divalent ionophore concentrations that may be employed can, e.g., be derived from available literature (e.g. see References [7]a) to e)) and commercial suppliers (e.g. eBioscience GmbH, Germany, article Nr. 00-4975-93 (in January 2017)). Ionomycin concentrations employed in the context of the present invention may, for example, be in a range of 1 to 700 µM, more preferably 200 to 700 µM, even more preferably 400 to 700 µM, even more preferably 600 to 700 µM, and most preferably 670 µM. A23187 concentrations employed in the context of the present invention may, for example, be in a range of 1 to 1400 µM, more preferably 400 to 1400 µM, even more preferably 800 to 1400 µM, even more preferably 1200 to 1400 µM, and most preferably 1340 µM.

The phorbol ester employed in the context of the present invention may be selected from different phorbol esters known in the art, preferably from any of the phorbol esters previously described in the context of T cell activation and/or expansion (i.e. phorbol esters that trigger activation and expansion of T cells). Phorbol esters that have been previously linked to T cell stimulation/activation are known in the art (e.g. see References [7]a) to e)). Accordingly, a phorbol ester employed in the present invention may, for example, be selected from the group consisting of phorbol 12-myristate-13-acetate (PMA), phorbol 12,13-didecanoate, phorbol 12,13-dibutyrate, and mezerin. The preferred phorbol ester to be employed is PMA (which is sometimes also referred to under the name 12-*O*-tetradecanoylphorbol-13-acetate (TPA), tetradecanoylphorbol acetate, or tetradecanoyl phorbol acetate). PMA is a diester of phorbol and a potent mitogen that is often employed in biomedical research. It is known to activate the signal transduction enzyme protein kinase C (PKC). A skilled person is aware that depending on the respective phorbol used, the concentration may need to be adapted. Non-limiting examples of phorbol ester concentrations that can be employed may e.g. be derived from the available literature (see e.g. References [7]a) to e) and commercial suppliers (e.g. eBioscience GmbH, Germany, article Nr. 00-4975-93 (in January 2017)). For example, PMA may be employed at a concentration range of 1 to 50 µM, more preferably 35 to 45 µM, and most preferably 40.5 µM.

As indicated above, step (i) of the method for T cell expansion of the present invention is culturing T cells in the presence of a divalent ionophore (preferably a Ca²⁺ ionophore) and a phorbol ester. In view of the preferences for divalent ionophores and phorbol esters discussed above, the most preferred combination to be employed is ionomycin and PMA. Accordingly, in a preferred embodiment, step (i) is culturing T cells in the presence of PMA and ionomycin. The combination of ionomycin and PMA triggers the IL-2 synthesis of T cells and the expression of its receptors on T cells through the activation of PKC and an increase of the cytosolic-free calcium concentration (see References 9).

However, in principle any of the combinations of a phorbol ester and a divalent ionophore other than PMA and ionomycin, such as phorbol 12,13-didecanoate and ionomycin, phorbol-12,13-dibutyrate and ionomycin, mezerin and ionomycin, PMA and A23187, phorbol 12,13-didecanoate and A23187, phorbol-12,13-dibutyrate and A23187, or mezerin and A23187 is expected to have similar synergistic effects and may thus be employed in the context of the present invention.

During step (i) of a method according to the present invention optionally one or more protein transport inhibitors may be present. A protein transport inhibitor promotes accumulation of secreted proteins and is believed to be beneficial for the method for T cell expansion of the present invention. Accordingly, step (i) of the method for T cell expansion may comprise or be: culturing a T cell in the presence of a phorbol ester, a divalent ionophore (preferably a Ca²⁺ ionophore), and one or more protein transport inhibitors. Preferably, at least two and even more preferably exactly two protein transport inhibitors are employed. Preferred protein transport inhibitors that may be employed in the context of the present invention are brefeldin A and/or monensin. Most preferably, two protein transport inhibitors being brefeldin A and monensin are employed. A skilled person is aware of typical concentrations of a protein transport inhibitor that has to be employed when culturing T cells. In particular, a skilled person is also aware that dependent on the respective protein transport inhibitor used the concentration may need to be adapted accordingly. Respective concentrations of protein transport inhibitors that can be employed may e.g. be derived from available literature (e.g. see References [7]f) to h)) and commercial suppliers (e.g. eBioscience GmbH, Germany, article Nr. 00-4975-93 (in January 2017)). For example, brefeldin A may be employed at a concentration range of 5 to 6 mM, more preferably 5,2 to 5,4 mM, and most preferably 5,3 mM. Monensin concentrations employed in the context of the present invention may, for example, be in a range of 0,9 to 1,1 mM, more preferably 0,95 to 1,05 mM, and most preferably 1 mM.

As evident from the above, in a particularly preferred embodiment of the method for T cell expansion step (i) may comprise or be: culturing a T cell in the presence of PMA, ionomycin, and the protein transport inhibitors brefeldin A and monensin. Notably, mixtures comprising PMA, ionomycin, and the protein transport inhibitors brefeldin A and monensin are also commercially available as a mixture dissolved in ethanol (Cell Stimulation Cocktail plus protein transport inhibitors 500X, eBioscience GmbH, Germany, article Nr. 00-4975-03 (in January 2017)). Accordingly in one aspect step (i) of the method of the present invention may also comprise culturing a T cell in the presence of a mixture comprising PMA, ionomycin, and the protein transport inhibitors brefeldin A and monensin (e.g. dissolved in ethanol). Said mixture may be a Cell Stimulation Cocktail plus protein transport inhibitors 500X (commercially available; e.g. from eBioscience GmbH, Germany). When employing a commercially available mixture, cells may be cultured in cell culture medium containing the Cell Stimulation Cocktail at the recommended dilution, for the times indicated herein elsewhere for step (i). E.g., cells may be cultured for 5 hours. Afterwards, cells may be centrifuged and suspended in culture medium without Cell Stimulation Cocktail and subjected to step (ii). In other words, culturing in the presence of a mixture comprising PMA, ionomycin, and the protein transport inhibitors brefeldin A and monensin (e.g. the Cell Stimulation Cocktail plus protein transport inhibitors 500X) may be employed as a prestimulation step before step (i).

Step (ii) of the method for T cell expansion according the present invention comprises or is culturing T cells in the presence of an agent capable of activating CD3 and/or an agent capable of activating CD28. In a preferred embodiment step (ii) may comprise or be culturing T cells in the presence of an agent capable of activating CD3 and an agent capable of activating CD28.

An "agent capable of activating CD3" may relate to any agent that activates CD3-dependent signalling pathways. CD3-dependent activation can be measured as described above for T cell activation, i.e. by detecting IL-2 and/or INF-γ levels, wherein an increase in IL-2 and/or INF-γ level upon addition of an agent indicates activation. Preferably, the agent capable of activating CD3 is capable of binding to/binds CD3. An agent capable of activating CD3 may also be referred to as a CD3-activating agent or as an agent that activates CD3-dependent signalling. Agents capable of activating CD3 are known in the art. In the context of the present invention the preferred agent capable of activating CD3 is an anti-CD3 antibody (e.g. an anti-human CD3 antibody), even more preferably an anti-CD3 antibody that stimulates/activates T cells upon binding, i.e. induces CD3-dependent signalling. Preferably, an anti-CD3 antibody which reacts with an epitope on the epsilon subunit within the CD3 complex in humans is used as CD3-activating agent. In principle, also any other peptide sequence mimicking antibody function, i.e. that binds to CD3 and activates CD3-dependent signalling could be employed. An anti-CD3 antibody may for example be selected from the group of commercially available antibodies consisting of: anti-human CD3 (purified) (eBioscience GmbH, Germany); (purified) anti-human CD3 antibody (BioLegend, US); anti-CD3 antibody, human, unconjugated (Abcam, US); monoclonal anti-CD3 antibody, human (purified) (Sigma-Aldrich, US); human CD3 epsilon antibody (unconjugated) (R&D Systems, US); CD3e monoclonal antibody, human (unconjugated) (Thermo Fisher Scientific, US); CD3 (purified), human (Immunotools GmbH, Germany); CD3 epsilon antibody (unconjugated), human (Novus Biologicals, US); CD3 antibody, human (unconjugated) (GeneTex, US); mouse anti human CD3 antibody (purified) (BioRad, US); (purified) anti-human CD3 antibody (Abgent, US); and (purified) mouse anti-human CD3 (BD Biosciences, US). Preferably, anti-human CD3 (functional grade purified), clone: OKT3 (eBioscience GmbH, Germany) is employed, which reacts with an epitope on the epsilon subunit within the CD3 complex in humans.

An "agent capable of activating CD28" may relate to any agent that activates CD28-dependent signalling pathways. Activation of CD28 can be measured as described above for T cell activation and/or the agent capable of activating CD3. Preferably, said agent is capable of binding to/binds to CD28. An agent capable of activating CD28 may also be referred to as a CD28-activating agent or as an agent that activates CD28-dependent signalling. Agents capable of activating CD28 are known in the art. In the context of the present invention the preferred agent capable of activating CD28 is an anti-CD28 antibody (e.g. an anti-human CD28 antibody). It is particularly envisaged that an anti-CD28 antibody that is capable of activating/activates CD28 signalling is employed. In principle, also any other peptide sequence mimicking antibody function, i.e. that binds to CD28 and activates CD28-dependent signalling could be employed. An anti-CD28 antibody may for example be selected from the group consisting of the following commercially available antibodies: anti-human CD28 (functional grade purified) (eBioscience GmbH, Germany); (purified) anti-human CD28 antibody (BioLegend, US); anti-CD28 antibody, human (unconjugated) (Abcam, US); monoclonal anti-CD28 antibody, clone CD28.2, human (purified) (Sigma-Aldrich, US); human CD28 antibody (unconjugated) (R&D Systems, US); CD28 monoclonal antibody (CD28.2), human (unconjugated) (Thermo Fisher Scientific, US); CD28, (purified) human (Immunotools GmbH, Germany); CD28 antibody (unconjugated), human (Novus Biologicals, US); CD28 antibody, human (unconjugated) (GeneTex, US); CD28 antibody, human (purified) (BioRad, US); (purified) anti-human CD28 (CD28.2) antibody (Abgent, US); and (purified) mouse anti-human CD28 (BD Biosciences, US). Preferably, anti-human CD28 (e.g. functional grade purified), clone 28.2 (eBioscience GmbH, Germany) is employed. The monoclonal antibody CD28.2 binds to human CD28.

A CD3-activating agent (e.g. an anti-CD3 antibody) employed in step (ii) is preferably attached to a surface. This particularly applies to a CD3-activating agent being an anti-CD3 antibody. A CD28-activating agent (e.g. an anti-CD28 antibody) employed in step (ii) may be provided soluble or attached to a surface. It is believed that a configuration in which the CD28-activating agent (e.g. an anti-CD28 antibody) is attached to a surface is particular advantageous. However, as demonstrated in the appended Examples and Figures, also a soluble CD28-activating agent (e.g. an anti-CD28 antibody) efficiently contributes to T cell activation and expansion. In particular, combining a CD3-activating agent (e.g. an anti-CD3 antibody) attached to a nanostructured surface with a soluble CD28-activating agent (e.g. an anti-CD28 antibody) results in a T cell activation and expansion that is superior compared to using a CD3-activating agent (e.g. an anti-CD3 antibody) alone and even compared to the state-of-the-art Dynabeads method provided that T cells are cultured (e.g. previously prestimulated) as described in step (i) of the method herein presented. Moreover, it is envisaged that using the same setting with a CD28-activating agent (e.g. an anti-CD28 antibody) being attached to a surface instead of being in solution may be at least similar if not even superior in performance (in particular when the surface is a nanostructured surface according to the present invention).

Accordingly, in a preferred embodiment step (ii) may be or comprise culturing a T cell in the presence of a CD3-activating agent (e.g. an anti-CD3 antibody) attached to a surface. In another preferred embodiment step (ii) may be or comprise culturing a T cell in the presence of a CD28-activating agent (e.g. an anti-CD28 antibody) attached to a surface. In a particular preferred embodiment step (ii) may be or comprise culturing a T cell in the presence of a CD3-activating agent (e.g. an anti-CD3 antibody) attached to a surface and a CD28-activating agent (e.g. an anti-CD28 antibody). The anti-CD28-activating agent may in this embodiment be provided soluble or attached to a surface. Accordingly, step (ii) may be or comprise culturing a T cell in the presence of a CD3-activating agent (e.g. an anti-CD3 antibody) attached to a surface and a soluble CD28-activating agent (e.g. an anti-CD28 antibody). Alternatively, step (ii) may be or comprise culturing a T cell in the presence of a CD3-activating agent (e.g. an anti-CD3 antibody) attached to a surface and a CD28-activating agent (e.g. an anti-CD28 antibody) attached to a surface.

If provided on a surface, a CD3-activating agent and/or CD28 activating agent may need to be attached in a certain orientation on said surface to be active depending on the agent used. For instance, an anti-CD3 antibody and an anti-CD28 antibody may need to be attached in a certain orientation that still allows binding of the anti-CD3 antibody to the CD3 and the anti-CD28 antibody to the CD28, respectively. Such an orientation is preferably an orientation in which the antibody constant region (F_{c}) faces the surface.

The "surface" to which a CD3-activating agent (e.g. an anti-CD3 antibody) and/or a CD28-activating agent (e.g. an anti-CD28 antibody) is attached may be a surface of a device or a support, e.g. a (substantially) planar surface (e.g. of a device), such as a nanostructured surface as described herein and in the appended Examples. Alternatively, the surface may also be the surface of a bead, e.g. the surface of a magnetic or non-magnetic bead (e.g. a commercially available Dynabead, e.g. available from Thermo Fisher Scientific, US, article Nr. 11131D (in January 2017)). Accordingly, in step (ii) of the method for T cell expansion of the invention a T cell may be incubated in presence of a bead (e.g. a commercially available Dynabead, e.g. available from Thermo Fisher Scientific, US, article Nr. 11131D (in January 2017)) to which a CD3-activating agent (e.g. an anti-CD3 antibody) and/or a CD28-activating agent (e.g. an anti-CD28 antibody) is attached. Preferred in the context of the present invention is a surface of a device, e.g. a (substantially) planar surface. The surfaces that may be employed are described herein or are known in the art, e.g. from References [6]. Particularly preferred is, however, to employ a device having a nanostructured surface as described herein. As illustrated in the appended Examples and Figures, using said device having a nanostructured surface allows for an even more efficient T cell expansion and activation than using Dynabeads, at least if used in the context of the advantageous method for expansion of T cells of the present invention.

An exemplary example how a device having a nanostructured surface may be employed is explained in detail in the appended Examples. In brief, a device having a nanostructured surface may be used as the bottom surface of a culturing well, e.g. by employing a chamber slide system and selecting the shape of the nanostructured surface as a slide that fits into the chamber. For instance, Lab-Tek chambers (8 wells, ThermoFisher Scientific, US) may be employed. However in principle any chamber slide systems and/or equivalent products may be employed. Notably, one may also only place a nanostructured surface in a conventional culturing vessel. For the method of the present invention the cells are then cultured according to the present invention on this surface at least during step (ii). The number of cells and the volume of media used for cultivation needs to be selected dependening on the size of the nanostructured surface/cultivation chamber. An illustrative example is provided by the appended Examples, in particular in Table 3.

Similarly, an example how state-of-the-art Dynabeads may be used is provided in the appended examples. In brief, the Dynabeads may be admixed with the T cells to be cultured and a suitable culture medium (as indicated elsewhere herein). The ratios of Dynabeads and T cells may be adjusted according to the manufacturer's protocol or as described in the appended Examples and Figures.

The term "attached to a surface" means that an agent is linked/immobilized to a surface, preferably stably linked/immobilized to a surface. The terms attached, linked, and immobilized are used interchangeably in the context of the present invention. In principle, an attachment may be a covalent bond or a non-covalent interaction. Particularly preferred are a covalent bond for RGD and a non-covalent interaction for antibodies, which e.g. allow the preferred orientations of antibodies (i.e. an orientation that allows antigen binding). The terms "attached" and "attachment" may alternatively also be referred to as "functionalized" and "functionalization" herein.

As indicated above, it is preferred in the context of the present invention that a surface on which the CD3-activating agent (e.g. an anti-CD3 antibody) and/or the CD28-activating agent (e.g. an anti-CD28 antibody) are attached is a nanostructured surface.

It is particularly envisaged that this nanostructured surface for attaching the activating agents employed in the context of the present invention is a surface that comprises titanium oxide. In a preferred embodiment the surface (preferably the surface on which the NPs of a nanostructured surface are placed) may comprise, consist of, or essentially consist of TiO₂. "Essentially consisting of" in this context means that at least 30%, preferably at least 40%, more preferably at least 50%, even more preferably at least 60%, even more preferably at least 70%, even more preferably at least 80%, even more preferably at least 90%, and most preferably at least 99% of the surface consist of TiO₂. A surface of the present invention may also be provided on top of a base structure or support, e.g. to form a device/object having said surface. Such a base structure or support may, for instance, be a glass slide. In a preferred embodiment, as also illustrated in the appended Example, a glass slide with a surface of TiO₂ can be employed. For generating such covered glass slides, e.g. sputtering or any other thin-film deposition method known in the art to generate such TiO₂ surfaces on glass slides can be used. A surface of TiO₂ may e.g. have a thickness of at least 200 nm. A thickness of at least 200 nm is believed to ensure that essentially the complete surface (e.g. at (about) 99% of the surface) is covered with TiO₂. This is an advantage because a TiO₂ surface is ideally suited for attaching RGDs comprising a phosphonic acid group thereto. The material of the base structure/support may not have necessarily the same properties to bind RGD. For instance, glass-phosphonic acid interactions are much less stable under aqueous conditions.

A nanostructured surface (of a device) used in the context of the method of the present invention may comprise NPs. In other words, the nanostructured surface may be formed by the above mentioned surface (e.g. consisting of or comprising TiO₂ and the NPs placed thereon (in particular the exposed surface of the NPs placed thereon (e.g. by BCML)). Preferably, an employed nanostructured surface (of a device) comprises NPs that are quasi-hexagonally ordered.

The order of the nanostructured surface and the interparticle distances can be calculated with a known software that assumes a six-closest-neighbor model (see Reference [10]) and gives a parameter from 0 to 1 to quantify the order, being 1 a perfectly hexagonal structure and 0 a completely random distribution. Quasi-hexagonally ordered means that the order parameter determined with this software is at least 0.5.

As discussed above, it is particularly envisaged that a CD3-activating agent (e.g. an anti-CD3 antibody) and/or a CD28-activating agent (e.g. an anti-CD28 antibody) employed in step (ii) of the method for T cell expansion of the present invention may be employed attached to a surface. If said surface is a nanostructured surface comprising NPs, preferably quasi-hexagonally ordered NPs, the CD3-activating agent (e.g. an anti-CD3 antibody) and/or the CD28-activating agent (e.g. an anti-CD28 antibody) employed in step (ii) are preferably attached to said NPs, most preferably exclusively to said NPs (i.e. not to the rest of the surface, which is referred to as interparticle space). In other words, in a preferred embodiment step (ii) of the method of the present invention may comprise or be culturing the T cell in the presence of a nanostructured surface comprising NPs, wherein said NPs (preferably the surface of the NPs, even more preferably the exposed surface of the NPs) are functionalized with a CD3-activating agent (e.g. an anti-CD3 antibody) and/or a CD28-activating agent (e.g. an anti-CD28 antibody). If the NPs are only functionalized with a CD3-activating agent (e.g. an anti-CD3 antibody), preferably a soluble CD28-activating agent (e.g. an anti-CD28 antibody) is provided.

Accordingly, in a preferred aspect, the present invention provides a method for T cell expansion comprising:
(i) culturing a T cell in the presence of a phorbol ester, a divalent ionophore, and optionally one or more protein transport inhibitors; and
(ii) culturing the T cell in the presence of an agent capable of activating CD3 and/or an agent capable of activating CD28, wherein said agent capable of activating CD3 and/or said agent capable of activating CD28 are attached (preferably exclusively attached) to the NPs of a nanostructured surface (of a device, support, or member).

A skilled person is aware that the nanostructured surface (e.g. of a support, member, or device) may comprise and/or consist of different materials and may be generated by different methods such as BCML, drop-casting, dip-pen nanolithography, nanoimprint lithography (NIL), or electron beam lithography to mention some (see References [11]). In the context of the present invention, it is particularly preferred that nanostructured surfaces are generated by BCML. Generating a nanostructured surface by BCML has the advantage that the nanoscale can be tightly controlled (see References [6]) in contrast to e.g. drop-casting. For example, the density of ligands attached to the NPs may be tightly regulated by varying the interparticle distance (i.e. the distance between NPs) using different block copolymers. As discussed further below, and shown in the appended Examples and Figures, selecting specific interparticle distance may improve T cell activation and expansion. At the same time, BCML is simpler, faster, and cheaper than other available lithographic methods.

Depending on the composition of the surface on which the NPs are placed during the fabrication of the nanostructured surface, a person skilled in the art will select a corresponding NP material that allows the fabrication of the required nanostructured surfaces. In the context of the present invention, it is particularly envisaged that the surface on which the NPs are placed comprises (preferably at least in the proportions indicated above) or preferably consists of TiO₂. Using such surfaces, in principle any NP that can be orthogonally functionalized when used on TiO₂ or a surface comprising TiO₂ can be used. For instance, AuNPs or silver nanoparticles (AgNPs) are NPs that can be orthogonally functionalized when used on a TiO₂ surface or a surface comprising TiO₂ (see References [12]). The nanostructured surface preferably comprises AuNPs and even more preferably quasi hexagonally ordered AuNPs.

Methods to attach agents such as antibodies to surfaces and in particular NPs are known in the art and may be employed to attach a CD3-activating agent and/or a CD28-activating agent on the surface and/or one or more NPs. Exemplary, activating agents being antibodies may be attached to a surface as, for example, described in Reference [6]c). In the context of the present invention, it is particularly envisaged that immobilizing activating agents to AuNPs or AgNPs (preferably AuNPs) of a nanostructured surface may comprise incubating the nanostructured surface in a solution of a thiolated nitrilotriacetic acid (e.g. HS-(CH₂)₁₁-(EG)₃-NTA which is e.g. available from Prochimia, Poland), e.g. at a concentration of 0.5 mM dissolved in an aqueous solution of 50 % ethanol for at least 1 h. NTA is a chelating agent and in aqueous solutions forms stable complexes with metal ions such as Ni²⁺. Subsequently, the nanostructured surface may be washed (e.g. with water) and may be equilibrated with a Hepes buffered saline (HBS) solution for e.g. 10 min. Afterwards, it may be immersed in a NiCl₂ solution (e.g. 10 mM) for e.g. 30 min, followed by an incubation in a solution of protein G with a polyhistidine tag consisting of 6 histidines (His6-protein G, BioCat GmbH, Germany; a tag comprising more than 6 histidines is also expected to work)), preferably a solution with a 10 µg/ml concentration of His6-protein G for e.g. 1 h. Then the nanostructured surface may be incubated with an antibody (e.g. at a concentration of 10 µg/ml) for e.g. 1 h. Finally, the nanostructured surfaces maybe (extensively) washed with HBS for 6 h at 4 °C under mild shaking.

The present inventors have shown that the average distance between two neighbouring NPs (referred to as interparticle distance) comprised on a nanostructured surface used in the context of a method of the present invention may influence T cell expansion and activation efficiency. The average distance between two neighbouring quasi-hexagonally ordered NPs may be from (about) 20 nm to (about) 150 nm, preferably from (about) 20 nm to (about) 100 nm, more preferably from (about) 20 nm to (about) 60 nm, more preferably from (about) 20 to 35 nm (e.g. (about) 35 nm, which offers easy production and high T cell activation and expansion). Preferably, the average is a median in this context. A neighbouring NP is the adjacent NP with the shortest distance to a NP. A distance is preferably measured between the central vertical axes of said NPs (preferably orthogonal to the surface). The (average) interparticle distance is proportional to the number of stimulating signals, i.e. the number(s) of CD3-activating agents (e.g. an anti-CD3 antibody) and/or CD28-activating agents (e.g. an anti-CD28 antibody) available to the cells and, thus, it is an important measure to determine the available stimulatory and/or costimulatory signals, respectively.

In the context of the method of the present invention, in step (ii) the T cell is cultured in the presence of a CD3-activating agent (e.g. an anti-CD3 antibody) and/or a CD28-activating agent (e.g. an anti-CD28 antibody), wherein said CD3-activating agent (e.g. an anti-CD3 antibody) and/or said CD28-activating agent (e.g. an anti-CD28 antibody) are preferably attached to a surface, preferably to a nanostructured surface (of a device/support/member), and most preferably exclusively to the NPs of the nanostructured surface. In a preferred embodiment a surface as employed in the present invention is further functionalized with a material that prevents (unspecific) protein adsorption. In a preferred aspect, said surface may be an adhesive surface. Preferably the surface (e.g. the adhesive and/or non-unspecificly protein adsorbing surface) may comprise a plurality of RGDs being attached to said surface. The RGD peptides are preferably attached to the part of the surface that is not covered with a CD3-activating agent (e.g. an anti-CD3 antibody) and/or a CD28-activating agent (e.g. an anti-CD28 antibody).

Accordingly, in the context of a nanostructured surface, RGD is preferably attached to the area of the surface between the NPs. The surface area between NPs may also be referred to as interparticle area or space. Preferably at least 50%, more preferably at least 60%, even more preferably at least 70%, even more preferably at least 80%, even more preferably at least 90%, and most preferably the complete interparticle area of a nanostructured surface is covered with RGD peptides.

RGD has been identified as a minimal fragment of the ECM protein fibronectin that may stimulate cell adhesion through integrin binding (see Reference [13]). However, RGD has not been employed in *ex vivo* T cell expansion and in particular not in T cell expansion with an anti-CD3 antibody and an anti-CD28 antibody. It is envisaged that RGD peptides contribute to the superior T cell activation and expansion capacity achieved with the method of the present invention by prolonging the duration of the T cell interaction with the CD3-activating agent (e.g. an anti-CD3 antibody) and/or a CD28-activating agent (e.g. an anti-CD28 antibody) that is/are attached to the surface (e.g. to the NPs of a nanostructured surface). Further, RGD peptides also contribute to avoid (non-specific) protein adsorption on the surface (e.g. a surface comprising TiO₂). Using an RGD background on the interparticle area of a nanostructured surface is believed to be superior to previously used backgrounds such as the protein-repellent PEG, which has been used in Matic et al. (see Reference [6]c)), because these structures do not allow for a prolonged interaction between T cells and the stimulatory and costimulatory agents on said surface.

An RGD peptide as employed in the context of the present invention may in principle be a tripeptide with the amino acid sequence RGD or may be a peptide (e.g. of more than 3 amino acids) that comprises an RGD tripeptide. Employing an RGD peptide of at most 5 amino acids is preferred in the context of the present invention. For example, an RGD peptide having a length of exactly 5 amino acids may be employed. More preferably, the RGD employed is a cyclic RGD. Cyclic RGD peptides are known in the art (see e.g. Reference [14]) and have the advantage of being more stable than the corresponding linear RGD peptides (see Reference [15]). The cyclic RGD employed in the context of the present invention has preferably a length of at least 5 amino acids. Even more preferably, the RGD has a length of exactly 5 amino acids. Further, an RGD (e.g. cyclic RGD) employed in the context of the present invention may also comprise an anchor group (also referred to as head group) and optionally a spacer group. An anchor group is a chemical moiety covalently linked to the RGD peptide that contributes or mediates the attachment of the peptide to a surface that is employed (e.g. a TiO₂ surface) in the context of the method of the present invention. A skilled person will be aware of which anchor needs to be selected for each surface material used. As indicated elsewhere herein, a preferred surface to be employed is a surface comprising, essentially consisting of, or consisting of TiO₂ (optionally with NPs thereon). For such a surface the preferred anchor group is one or more phosphonic acid groups (see Reference [16]). A spacer group is a structure that provides a covalent link between the anchor and the peptide. A skilled person will be aware whether a spacer group is necessary and if necessary, which spacer needs to be selected for a given system (e.g. a PEG linker). Accordingly, in the context of the present invention it is particularly preferred to use an RGD peptide comprising a phosphonic acid as anchor group and aminohexanoic acid groups as spacer. Even more preferably, a cyclic RGD peptide (e.g. being at least of 5 amino acids or exactly 5 amino acids in length) comprising a phosphonic acid as anchor group and aminohexanoic acid groups as spacer are employed. RGD peptides such as cyclic RGD peptides comprising a phosphonic acid as an anchor group and aminohexanoic acid groups as spacer are known in the art and are, for example, described in Reference [14] (e.g. the peptide referred to as "peptide 1:RGD-spacer-tetraphosphonate" in Reference [14], which is herewith incorporated in its entirety). Thus, a particularly preferred RGD employed in the context of the present invention may have the following formula:

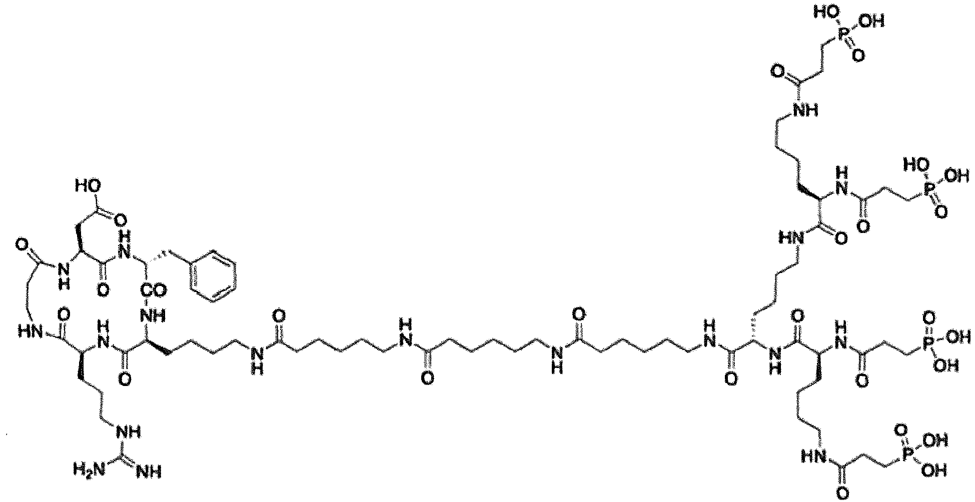

This preferred RGD peptide comprises the RGD sequence motif as a part of a cyclic peptide of 5 amino acids in length (left part), a phosphonic acid as an anchor group (right side) and aminohexanoic acid group as spacer (see middle part). Employing RGD peptides comprising a phosphonic acid as an anchor group, e.g. cyclic peptides comprising a phosphonic acid as an anchor group, is advantageous if a surface comprising, essentially consisting of, or consisting of TiO₂ is used in the method of the present invention. This combination has the advantage that RGD can be efficiently attached to the surface via its phosphonic acid group. The attachment has been found to be particularly stable also under aqueous conditions, e.g. when culturing cells in the presence of cell culture medium. Accordingly, in the described context, phosphonic acid groups are advantageous over e.g. the widely used silanes that are used to functionalize oxides because this R-Si-O attachment is much more water sensitive and unstable.

A person skilled in the art is aware of methods and procedures to generate surfaces having an adhesive background, such as RGD peptides (e.g. a cyclic RGD peptide). The process of attaching a background such as an RGD is also known as the functionalization of a surface. Exemplary, an RGD (e.g. a cyclic RGD) comprising a phosphonic acid as an anchor group may be attached to a surface comprising or consisting of TiO₂ as described in the Examples. In brief, nanostructured surfaces may be incubated with a 25 µM aqueous solution of a cyclic RGD that contains phosphonic acids for 12 h at room temperature (RT; e.g. 20°C).

The term "T cell" as used in the context of the present invention may in principle include any T cell known in the art. T cells known in the art express CD3, i.e. are CD3+ (wherein the "+" indicates that the respective surface marker protein is expressed in these cells) and can thus be identified via this surface marker using well known state of the art methods such as flow cytometry using anti-CD3 antibodies. T cells known in the art include, for example CD4+ cells and CD8+ cells. Preferably, a T cell employed in the context of the present invention is a CD4+ T cell (alternatively referred to as T helper cell or CD4-positive T cell), i.e. a T cell that expresses CD4 on its surface. Accordingly, preferably a method for T cell expansion as described herein may be a method for expansion of CD4+ T cells. A T cell employed in the present invention may be of any origin, but it is preferred to employ a human T cell in order to provide human T cells for adoptive cell therapy. The T cell employed in the context of the present invention can in principle be a T cell that has been previously cultured or a T cell that has been freshly isolated by methods known in the art. In other words, the T cells employed may be certain cell lines or primary cells. Preferred is the use of primary T cells. Most preferred is the use of primary T cells that have been isolated and not previously cultured.

In principle, a single type of T cells, a subgroup of different subtypes of T cells, or even a mixture of T cells with one or more unknown T cell subtypes may be subjected to the method for T cell expansion of the present invention. A T cell may be isolated before being subjected to the method for expansion by methods known in the art (e.g. the MACS Cell Separation (Miltenyi Biotec, Germany), which is based on the specific surface markers of the respective T cell type known in the art). Alternatively, also a mixture of different types of T cells that has been separated from other cell types may be employed. Actually, it is even envisaged that T cells can be cultured in a mixture with other cells.

Methods for preparing and/or isolating primary T cells from different sources such as blood, lymph nodes, or spleen are well known in the art. Similarly also methods for isolating certain subtypes of T cells (e.g. CD4+ T cells) are well known in the art and may be based on fluorescence-activated cell sorting (FACS) and/or magnetic bead-based cell sorting technologies, e.g. commercially available technologies such as the MACS cell separation systems (Miltenyi Biotec, Germany). The basic principle underlying these technologies is to sort cells depending on the surface markers expressed. Using these methods highly pure T cell populations can be generated and employed in the context of the present invention. For many T cell subtypes even commercial kits for isolation/purification are available such as the CD4+ T Cell Isolation Kit, human (Miltenyi Biotec, Germany). The term isolated/purified T cell subtype as used herein refers to a T cell population in which at least 90% and preferably at least 95% of the cells have the subtype specific markers. Exemplary, a method for isolating primary CD4+ T cells from a blood sample (e.g. a human blood sample) may be performed as follows. First, a density gradient centrifugation to recuperate the peripheral blood mononuclear cells (PBMCs) from the blood sample may be performed. Then, the needed amount of CD4+ T cells may, for example, be obtained using a commercially available kit (CD4+ T Cell Isolation Kit human, Miltenyi Biotec, Germany), which is based on a magnetic purification. Briefly, all non-CD4+ T cells are marked with anti-biotin, which further react with magnetic beads that stay in the chromatography column placed on a magnet. Thus, only CD4+ T cells pass through the column and CD4+ T cells can be suspended in medium (e.g. the RPMI Medium 1640 Glutamax (Thermo Fisher Scientific, US) with 10% fetal bovine serum (FBS), and 1% penicillin/streptomycin (P/S) as used in the Example of the present invention).

Step (i) as well as step (ii) of the method for T cell expansion comprise culturing T cells. Culturing conditions and media suitable for cultivation of T cells are well known in the art and can readily be selected by a person skilled in the art (e.g. see Reference [6]c)). For example, culturing cells in an incubator with a 5% CO₂ at 37 °C using RPMI Medium 1640 Glutamax + 10% FBS + 1% P/S are suitable conditions.

The duration of the culturing in step (i) of the method of the present invention may preferably be at least 1 hour, more preferably at least 2 hours, even more preferably at least 3 hours, and most preferably at least 5 hours. Further, the duration of the culturing in step (i) of the method of the present invention may preferably be at most 12 hours, more preferably at most 10 hours, even more preferably at most 7 hours, and most preferably at most 5 hours. Accordingly, The duration of the culturing in step (i) of the method of the present invention may be from 1 to 12 hours, preferably from 3 to 10 hours, more preferably from 3 to 7 hours, and most preferably 5 hours.

The duration of the culturing in step (ii) of the method of the present invention may preferably be at least 12 h, more preferably at least 1 day, even more preferably at least 3 days, and most preferably 7 days. After e.g. 7, 8, 9, or 10 days of culturing in step (ii) the employed activating agents may be refreshed, e.g. by providing a new surface/beads.

In a second aspect the present invention provides for a device, preferably a device for T cell activation and/or expansion/proliferation. The device may be a device for T cell expansion. In principle, the device may also be a device for merely T cell activation. The device according to the present invention may comprise an RGD peptide, preferably a plurality of RGD peptides, attached to the upper surface of the device. Providing a device comprising RGD attached to a surface of the device allows for providing an adhesive surface. Accordingly, the present invention can, for instance provide for a device such as a device having a nanostructured surface for *ex vivo* T cell expansion that is adhesive. Providing an RGD background on a surface of the device facing the cells to be cultured enables to increase the time of interaction between the cells and the surface. Thereby, e.g. the contact of the cells with stimulatory or costimulatory agents that may be simultaneously provided on the surface comprising an RGD background can be increased. The present inventors have found that this is particularly advantageous for promoting the efficacy of T cell expansion.

RGD has been previously described to induce adhesion and spreading of adherent cells such as fibroblasts or osteoblasts, mimicking cell-extracellular matrix (ECM) interactions *in vitro.* However, employing RGD in the context of *ex vivo* primary T cell activation and expansion has not been described before. In fact, the use of RGD peptides with T cells has only been described in the context of multifunctional nanorods that serve as nanobridges to modulate T cell-mediated immunity of a Jurkat T cell line (see Reference [17]). The RGD peptides employed in Reference [17] were linear and comprised carboxylic acids as head groups. Furthermore, the RGD was not even part of the activation system per se, which consists of dendritic cells in Reference [17]. Even though it has been described that T cells interact with the ECM *in vivo* through integrin receptors, which are known to recognize RGD, a skilled person would have had no indication that RGD could have an advantageous technical contribution in *ex vivo* T cell activation and expansion. This is due to the fact that for *ex vivo* T cell activation and expansion the IS formed by a T cell and an APC is simulated and the prior art teaches that this interface relies on the LFA-1/ICAM-1 integrin-ligand interaction, which is believed to mimic the T cell-APC interface. A contribution of the interaction of integrins and RGD containing peptides to the T cell-APC interface and/or the *in nitro* mimicking of said interface does not seem to have been considered yet, in particular not in the context of devices having surfaces functionalized with other T cell activating agents. Thus, it could have not been expected by a person skilled in the art that a device of the present invention comprising a surface being covered with an RGD has the advantageous technical contribution with respect to T cell expansion and activation, especially for primary T cells which are the ones that are used in adoptive cell therapy and have medical applications, as described herein.

To increase the long-term maintenance of the positive effect of the RGD during culturing, it is preferably envisaged that the device of the present invention has a (upper) surface comprising TiO₂ and an RGD comprising a phosphonic acid attached/linked (preferably covalently) thereto. Compared to the attachment of other coatings, such as poly(ethylene glycol) (PEG) on glass surfaces via silane groups (as described in Reference [6]c) or the linkage of the RGD peptide to the nanorods of Reference [17], this link has the advantage of being much more stable under aqueous conditions, e.g. in the presence of cell culture medium. Therefore the link between the surface of a device according to the present invention and RGD is very stable under aqueous conditions, which is believed to be beneficial for efficient T cell activation and expansion. It may further increase the long-term stability of the device for T cell activation and expansion and therefore reduce the costs of T cell expansion. Stability may further be increased by employing circular RGD peptides with the above-mentioned characteristics. Accordingly, the present invention provides a highly advantageous device (for T cell expansion) that is ideally suitable for *in vitro* cell culture experiments, in particular for T cell activation and expansion.

The dimensions of the nanostructured surface of the device according to the present invention may be selected depending on the amount of cells intended to be cultivated. For instance, the nanostructured surface may be selected to have an area of (about) 1 cm² per 0.25x10⁶ to 0.5x10⁶ cells.

It is particularly envisaged that a device according to the present invention is a device having a nanostructured surface, preferably a device having a nanostructured surface for *ex vivo* T cell activation and expansion.

The (upper) surface of the device to which an RGD is linked may in principle be formed of any material suitable to attach a peptide or a peptide derivative. Preferably the (upper) surface of said device (preferably a device having a nanostructured surface) comprises TiO₂. In a preferred embodiment the (upper) surface may essentially consist of or consist of TiO₂. Accordingly, in a preferred embodiment the present invention relates to a device (preferably a device having a nanostructured surface), wherein RGD is attached to the surface of said device, wherein said surface comprises, essentially consist of, or consists of TiO₂. "Essentially consisting of" in this context means that at least 30%, preferably at least 40%, more preferably 50%, even more preferably 60%, even more preferably at least 70%, even more preferably at least 80%, even more preferably at least 90%, and most preferably at least 99% of the surface consist of TiO₂.

The (upper) surface of the device of the present invention may also be provided on top of a base structure. Such a base structure may, for instance, be formed from borosilicate glass (e.g., may be a glass slide), quartz, or a silicon wafer. In a preferred embodiment, as also illustrated in the appended Example, a glass slide with a (top) surface of TiO₂ can be employed. For generating a surface comprising, essentially consisting of, or consisting of TiO₂ on a glass slide, e.g. sputtering or any other thin-film deposition method known in the art to generate such TiO₂ surfaces on glass slides can be used. Methods for generating a surface comprising, essentially consisting of, or consisting of TiO₂ on a glass slide are, for example, described in the "Handbook of Thin Film Deposition. Techniques, Processes, and Technologies" (edited by Krishna Seshan; Elsevier Inc., 3rd Edition; Waltham (US); 2012). A surface of TiO₂ may preferably have a (nominal) thickness of at least, about 200 nm, or 200 nm. The advantages of said thickness are described further above in the context of the method of the present invention.

The device of the present invention may comprise an upper and a lower layer, wherein the upper layer forms the upper surface and comprises, essentially consists of, or consists of TiO₂. The lower layer may be formed by a different material such as borosilicate glass, quartz, or silicon. The material forming the lower layer needs to be suitable to be (stably) fixed with the upper layer, e.g. the top layer may be a coat on the lower layer. For instance, it is envisaged that the device according to the present invention has a lower layer being a glass slide, which is covered with an upper layer comprising, essentially consisting of, or consisting of TiO₂. Such upper layer may be placed on the lower layer by methods known in the art, e.g. sputtering.

RGD peptide(s) attached to the surface of the device include(s) that the RGD peptide(s) is/are covalently or non-covalently bound to the surface. The non-covalent interaction is an electrostatic interaction. Preferred is a covalent binding. It is also envisaged that the attachment of RGD to the (upper) surface of the device will be resistant to aqueous solutions since the device of the present invention is intended for cell culture applications, which typically employ aqueous media. Resistant in this context may mean that after an incubation of 24 h of the (upper) surface of the device of the present invention in the presence of an aqueous solution, preferably a standard cell culture medium (e.g. a T cell culture medium), at 37°C, at least 80%, preferably at least 90%, and most preferably at least 99% of the RGD peptides remain bound to the surface.

As mentioned above, it is particularly preferred that the device of the present invention is a device having a nanostructured surface. A nanostructured surface comprises by definition NPs. These NPs may be placed on a surface in distinct geometrical configurations so as to form a nanostructured surface. While in principle the NPs of a nanostructured surface of the present invention may have any geometrical configuration, in a preferred aspect it is envisaged that the nanostructured surface according to the present invention comprises quasi-hexagonally ordered NPs. An advantage of quasi-hexagonally ordered NPs is the simplicity of producing it by techniques such as BCML. Specifically, the quasi-hexagonal array is obtained due to the closed-packed configuration that the micelles form in the coating process. Even more preferably, essentially all, or all NPs of the nanostructured surface of the present invention are ordered hexagonally. "Essentially all" in this context means that at least 50%, preferably at least 60%, more preferably at least 70%, even more preferably 80%, even more preferably 90%, and most preferably 99% of the NPs of said nanostructured surface are hexagonally ordered.

A nanostructured surface of the device may be formed by the surface (e.g. a TiO₂ surface) and the exposed surface of the NPs placed thereon (e.g. by BCML).

The (average) distance between two neighbouring quasi-hexagonally ordered NPs (also referred to as interparticle distance) may be from (about) 20 nm to (about) 150 nm, preferably from (about) 20 nm to (about) 100 nm, more preferably from (about) 20 nm to (about) 60 nm, and most preferably (about) 20 to (about) 35 nm (e.g. (about) 35 nm, which is easy to fabricate and advantageous for T cell expansion). Preferably, the average is a median in this context. A neighbouring NP is the adjacent NP with the shortest distance to a defined NP. A distance is preferably measured between the central vertical axes of two neighbouring NPs. The interparticle distance influences the density of molecules such as T cell stimulatory agents that can be attached to the surface of the device according to the present invention. The present inventors have found that the interparticle distances defined are advantageous for T cell activation and expansion using a T cell stimulatory agent (e.g. a CD3-activating agent such as an anti-CD3 antibody) and/or a T cell costimulatory agent (e.g. a CD28-activating agent such as an anti-CD28 antibody) attached to the NPs (e.g. the exposed NP surface). As illustrated in the appended Examples and Figures, an interparticle distance of about 35 nm turned out to be particularly beneficial for T cell expansion.

NPs of a device (preferably a device having a nanostructured surface) according to the present invention may consist of various materials known in the art. Particularly preferred in the context of the present invention are AuNPs or AgNPs. Most preferred are AuNPs. Accordingly, the device of the present invention is preferably a device having a nanostructured surface, wherein said nanostructured surface comprises of a TiO₂ surface comprising quasi-hexagonally ordered NPs fixed thereto (preferably AuNPs), and wherein a plurality of RGD peptides are attached to said TiO₂ surface.

As mentioned above, a device according to the present invention is preferably a device having a nanostructured surface. The surface of a nanostructured surface to which the RGD peptide(s) is/are attached is preferably only the surface between the NPs of the nanostructured surface (i.e. the interparticle area) or a portion thereof (e.g. at least 50%, preferably at least 60%, even more preferably at least 70%, even more preferably at least 80%, even more preferably at least 90%, and most preferably at least 99% of the interparticle area). The NPs themselves are preferably not covered by RGD.

An RGD peptide as employed in the context of the device of the present invention may in principle be a tripeptide sequence or may be comprised of a longer peptide sequence. However, employing an RGD peptide of at most 5 amino acids is particularly preferred in the context of the present invention. For example an RGD peptide having exactly a length of 5 amino acids may be employed.

It is particularly also envisaged that an RGD peptide employed in the context of the device according to the present invention is a cyclic RGD peptide. Cyclic RGD peptides are known in the art (see e.g. Reference [14]) and have the advantage of being more stable than the corresponding linear RGD peptides such as, e.g., the one used in the context of the nanorods of Reference [17]. A cyclic RGD peptide may preferably comprise at least 5 amino acids or exactly 5 amino acids. Preferably, an RGD (e.g. a cyclic RGD) employed in the context of the present invention may also comprise an anchor group and a spacer group. An anchor group refers to a chemical group that is covalently linked to the RGD peptide that contributes or mediates the attachment of the peptide to the surface of the device (e.g. a TiO₂ surface). A skilled person will be aware of which anchor needs to be selected for each surface material used. As indicated elsewhere herein, a preferred surface of the device according to the present invention is a surface comprising, essentially consisting of, or consisting of TiO₂. For such a surface the preferred anchor is one or more phosphonic acid groups (see Reference [16]). A spacer group is a structure that provides a covalent link between the anchor and the peptide. A skilled person will be aware of which spacer needs to be selected for a given system (e.g. a PEG linker). Accordingly, it is particularly preferred to use an RGD peptide comprising a phosphonic acid as anchor group and aminohexanoic acid groups as spacer in the context of the present invention. Even more preferably, a cyclic RGD peptide comprising a phosphonic acid as anchor group and aminohexanoic acid groups as spacer are employed. RGD peptides such as cyclic RGD peptides (e.g. comprising at least 5 or exactly 5 amino acids) comprising a phosphonic acid as an anchor group and aminohexanoic acid groups as spacer are known in the art and are, for example, described in Reference [14] (e.g. the peptide referred to as "peptide 1:RGD-spacer-tetraphosphonate" in Reference [14] which is herewith incorporated in its entirety). Thus, a particularly preferred RGD employed in the context of the device of the present invention may have the following formula:

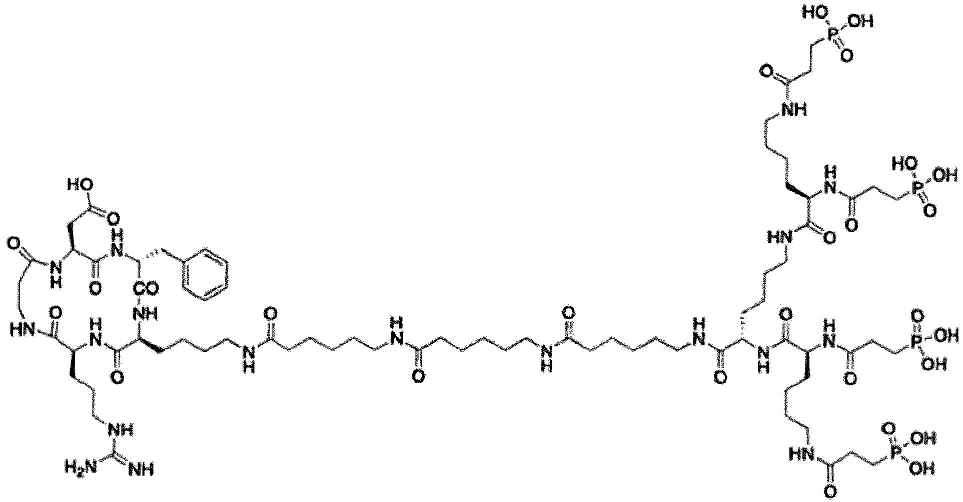

This preferred RGD comprises RGD peptides comprising the RGD as a part of a cyclic peptide of 5 amino acids in length (left part), a phosphonic acid as an anchor group (right side) and aminohexanoic acid group as spacer (see middle part). Employing RGD peptides comprising a phosphonic acid as an anchor group such as cyclic peptides comprising a phosphonic acid as an anchor group (e.g. see formula above) is advantageous when a surface comprising, essentially consisting of, or consisting of TiO₂ is used in the method of the present invention. This combination has the advantage that RGD can be efficiently attached to the surface via its phosphonic acid groups that interact with the TiO₂. The attachment has been found to be particularly stable also under aqueous conditions, e.g. when culturing cells in the presence of cell culture medium. Accordingly, in the described context, phosphonic acid groups are advantageous as regards to their stability in aqueous solutions over e.g. the widely used silanes that are used to functionalize oxides.

A person skilled in the art is aware which methods and procedures may be employed to generate surfaces with RGD peptides (e.g., cyclic RGD peptides) attached thereto. Corresponding methods are also disclosed herein, e.g. in the appended Examples and Figures. A process of attaching a background such as an RGD peptide is also known as functionalization or immobilization of the background molecule on a surface of a surface. Exemplary, an RGD peptide (e.g. a cyclic RGD peptide) comprising a phosphonic acid as an anchor group may be attached to a surface comprising or consisting of TiO₂ as described in the Example. In brief, nanostructured surfaces may be incubated with a 25 µM aqueous solution of a cyclic RGD peptide that contains phosphonic acids for 12 hours at room temperature (RT; e.g. 20°C).

The device according to the present invention is preferably a device for T cell activation and/or expansion. Accordingly, the device according to the present invention may further comprise an agent for stimulating T cells and/or an agent for costimulating T cells. Stimulating means an agent triggering T cell receptor signaling. Costimulating refers to costimulating signals promoting T cell activation. Preferably, the device according to the present invention comprises an agent capable of activating CD3 (a CD3-activating agent) and/or an agent capable of activating CD28 (a CD28-activating agent). Most preferably the device according to the present invention comprises an agent capable of activating CD3 (a CD3-activating agent) and an agent capable of activating CD28 (a CD28-activating agent). An agent capable of activating CD3 is a stimulatory agent. An agent capable of activating CD3 provides a stimulatory signal mimicking the T cell receptor signaling. An agent capable of activating CD28 provides a costimulatory signal for T cell activation.

What has been said with respect to an "agent capable of activating CD3" in context of the method of the invention, above, applies to the device of the invention *mutatis mutandis.*

Similarly, what has been said with respect to an "agent capable of activating CD28" in context of the method of the invention, above, applies to the device of the invention *mutatis mutandis.*

While it is preferred that the device according to the present invention further comprises a CD28-activating agent attached to the (upper) surface (see above) it is also encompassed by the present invention that the comprised CD28 activating agent is not attached to the surface but provided in solution together with the device. As illustrated in the appended Example such configuration results in a particularly high efficacy of T cell activation and expansion.

As mentioned above, when the device of the present invention is a device having a nanostructured surface, the surface to which the RGD peptide(s) is/are preferably attached to is the interparticle area of the (upper/nanostructured) surface. When the device further comprises a CD3-activating agent (e.g. an anti-CD3 antibody) attached to the (upper/nanostructured) surface, this CD3-activating agent (e.g. an anti-CD3 antibody) is preferably attached to the NPs (preferably to the surface of the NPs protruding from the upper direction), most preferably exclusively from the NPs (i.e. not from the interparticle area). Similarly, when the device further comprises a CD28-activating agent (e.g. an anti-CD28 antibody) attached to the (upper/nanostructured) surface, this CD28-activating agent (e.g. an anti-CD28 antibody) is preferably attached to the NPs (preferably to the surface of the NPs protruding from the upper direction), most preferably exclusively from the NPs (i.e. not from the interparticle area). Moreover, when the device further comprises a CD3-activating agent (e.g. an anti-CD3 antibody) and a CD28-activating agent (e.g. an anti-CD28 antibody) attached to the (upper/nanostructured) surface, the CD3-activating agent (e.g. an anti-CD3 antibody) and the CD28-activating agent (e.g. an anti-CD28 antibody) are preferably attached to the NPs (preferably the surface of the NPs protruding from the upper direction), most preferably exclusively from the NPs (i.e. not from the interparticle area).

Accordingly, in a preferred aspect, the device of the present invention is a device having an (upper) nanostructured surface comprising quasi-hexagonally ordered NPs and an interparticle area between said NPs, wherein an RGD is attached to the interparticle area of said upper nanostructured surface but not attached to said NPs, and wherein a CD3-activating agent (e.g. an anti-CD3 antibody) is attached to most or preferably all of said NPs but not to the interparticle area. In an even more preferred aspect the device may further comprise a CD28-activating agent (e.g. an anti-CD28 antibody) that is attached to one or preferably more of the NPs but not the interparticle area.

The term "attached to a surface" as used in the context of the CD3-activating agent and/or the CD28-activating agent means that an agent is linked/immobilized to a surface, preferably stably linked/immobilized to a surface. In principle, such link may be a covalent bond or a non-covalent interaction. Particularly preferred is an interaction that enables the preferred orientation of the antibodies (e.g. that the antigen binding regions are free and can bind to antigens). This orientation can be achieved e.g. with a non-covalent interaction. The term attachment may alternatively also be referred to as functionalization or immobilization herein. A skilled person is aware of methods to attach a CD3-activating agent (e.g. an anti-CD3 antibody) and/or a CD28-activating agent to a surface, in particular to NPs. For instance, agents being antibodies may be attached to surfaces such as the surface of NPs as described in Reference [6]c). As described herein and in the appended Example, attaching antibodies to AuNPs or AgNPs (preferably AuNPs) of a nanostructured surface may be achieved by incubating a device with a nanostructured surface (e.g. a TiO₂ surface with AuNPs) in a 0.5 mM solution of a thiolated nitrilotriacetic acid (e.g. HS-(CH₂)₁₁-(EG)₃-NTA e.g. available from Prochimia, Poland) dissolved in an aqueous solution of 50 % ethanol for at least 1 h. NTA is a chelating agent and in aqueous solutions forms stable complexes with metal ions such as Ni²⁺. Subsequently, the device/nanostructured surface may be washed with water and equilibrated with an HBS solution for 10 min. Afterwards, it is immersed in a 10 mM NiCl₂ solution for 30 min, followed by an incubation in a 10 µg/ml solution of a His6-protein G (BioCat GmbH, Germany; a tag comprising more than 6 histidines is also expected to work) for 1 h. Protein G is coupled to the NTA by means of a coordination complex with Ni²⁺ through its histidine tag. Moreover, protein G is an immunoglobulin-binding protein that binds the Fc region of antibodies ensuring their site-directed attachment and therefore enabling maximal biological activity. Then the device/nanostructured surface is incubated with an antibody to be attached (e.g. at a concentration of 10 µg/ml) for 1 h. Finally, the device/nanostructured surface is extensively washed with HBS for 6 h at 4 °C under mild shaking.

As discussed above, the present invention provides a method for T cell expansion and a device (for T cell expansion). It is of course also envisaged that in any of the embodiments of the method for T cell expansion, a surface comprising the characteristics of the surface (e.g. the nanostructured surface) of a device of the present invention or preferably the device of the present invention may be employed.

The present invention further provides a method for producing the device according to the present invention as defined in any of the embodiments herein. Respective steps of a method of production are discussed in the context of describing the different features of a device of the present invention. A method of producing a device according to the present invention may comprise any combination of these steps. A preferred embodiment for a method for producing a device according to the present invention is provided in the appended Example.

A method for producing a device according to the present invention may comprise providing a device and attaching an RGD peptide (e.g. a cyclic RGD peptide) to a surface of said device. Preferably, the method of production may comprise providing a nanostructured surface comprising NPs (e.g. AuNPs or AgNPs) and attaching an RGD peptide.

What has been said for how an RGD peptide can be attached to a device of the present invention and/or the preferences of RGD peptides to be used of course applies *mutatis mutandis* for the method of producing a device according to the present invention.

Accordingly, attaching an RGD peptide to the surface is preferably achieved when an RGD peptide comprising a phosphonic acid is employed and the surface comprises TiO₂. As discussed above, this has the advantage that the attachment of the RGD to the surface is stable under aqueous conditions such as in the presence of cell culture medium. In other words the method for producing a device according to the present invention may comprise providing a device having a (nanostructured) surface comprising TiO₂ and incubating said surface or parts thereof with a solution comprising an RGD peptide (e.g. a cyclic RGD peptide) to attach said RGD peptide (e.g. the cyclic RGD peptide) to said surface. In a preferred embodiment said device is a nanostructured surface and the RGD is only attached to the interparticle space of said nanostructured surface. An exemplary example of how RGD maybe bound to a (nanostructured) surface is described in the appended Example. Accordingly, the step of attaching an RGD to a surface or parts thereof may involve incubating a (nanostructured) surface or parts thereof with an aqueous solution of an RGD that contains phosphonic acids (e.g. for 12 h) at RT, wherein said surface comprises, essentially consist of, or consists of TiO₂. The definition of essentially consists of TiO₂ as provided elsewhere herein applies *mutatis mutandis.*

Said method for the production of a device according to the present invention may further comprise immobilizing a CD3-activating agent (e.g. an anti-CD3 antibody) to the surface or parts thereof of the device. Preferably, when the device has a nanostructured surface comprising NPs (e.g. AuNPs or AgNPs), the CD3-activating agent (e.g. the anti-CD3 antibody) may be attached to the surface of the NPs of said nanostructured surface but not to the area between the NPs (interparticle space). Further, the method of production may also comprise immobilizing a CD28-activating agent (e.g. an anti-CD28 antibody) to the surface of the device. Preferably, when a nanostructured surface comprising NPs (e.g. AuNPs or AgNPs) is employed the CD28-activating agent (e.g. the anti-CD28 antibody) may be attached to the surface of the NPs of said nanostructured surface but not to the area between the NPs (interparticle space). The attachment of agents to the surface may alternatively also be referred to as functionalization with the respective agents.

A skilled person is aware of methods to attach a CD3-activating agent (e.g. an anti-CD3 antibody) and/or a CD28-activating agent (e.g. an anti-CD28 antibody) to a surface, in particular to NPs (e.g. AuNPs or AgNPs). A method for binding an agent being an antibody to a surface such as the surface of NPs is, for example, described in Reference [6]c). In the context of the present invention it is particularly envisaged that immobilizing activating agents to AuNPs or AgNPs (preferably AuNPs) of a nanostructured surface (of a device) may comprise incubating the nanostructured surface (of the device) in a solution of a thiolated nitrilotriacetic acid (e.g. HS-(CH₂)₁₁-(EG)₃-NTA from Prochimia, Poland), e.g. at a concentration of a 0.5 mM, dissolved in an aqueous solution of 50 % ethanol for e.g. 1 h. NTA is a chelating agent and in aqueous solutions forms stable complexes with metal ions such as Ni²⁺. Subsequently, the nanostructured surface may be washed (e.g. with water) and may be equilibrated with a HBS solution for e.g. 10 min. Afterwards, it may be immersed in a NiCl₂ solution (e.g. 10 mM) for e.g. 30 min, followed by an incubation in a solution of a His6-protein G (BioCat GmbH, Germany; of course also a tag comprising more than 6 histidines would work)), preferably a solution with a 10 µg/ml concentration of a His6-protein G for e.g. 1 h. Then the nanostructured surface may be incubated with an antibody to be attached (e.g. at a concentration of 10 µg/ml) for e.g. 1 h. Finally, the nanostructured surface may be (extensively) washed with HBS for 6 h at 4 °C under mild shaking. It is particularly advantageous to use a HBS buffer (instead of a PBS buffer as described in Reference [6]c)) during the functionalization process described above. This has the advantage that the risk for detachment of RGD (e.g. cyclic RGD) comprising phosphonic acid group(s) from a TiO₂ surface (if employed) is minimized. If a PBS buffer was used instead, the PBS salts could substitute the phosphonic acid group(s) of the RGD (e.g. cyclic RGD).

In yet another aspect, the present invention relates to the use of any of the devices of the present invention for activating and expanding T cells described herein. Of course in this aspect, any of the embodiments and definitions described in the context of the device of the present invention applies *mutatis mutandis.*

In a preferred aspect the use of a device according to the present invention may further comprise the use of a phorbol ester (e.g. PMA), a divalent ionophore (e.g. ionomycin), and optionally one or more protein transport inhibitors (e.g. brefeldin A and/or monensin). In other words, the use according to the present invention may comprise the use of a device according to any of the embodiments disclosed herein which further comprises a phorbol ester (e.g. PMA), a divalent ionophore (e.g. ionomycin), and optionally one or more protein transport inhibitors (e.g. brefeldin A and/or monensin).

What has been said elsewhere herein (e.g. in the context of the method of the present invention) with respect to a divalent ionophore to be employed applies *mutatis mutandis* to the uses disclosed herein.

Further, what has been said elsewhere herein (e.g. in the context of the method of the present invention) with respect to a phorbol ester to be employed applies *mutatis mutandis* to the uses disclosed herein.

Similarly, what has been said elsewhere herein (e.g. in the context of the method of the present invention) with respect to protein transport inhibitor(s) to be employed applies *mutatis mutandis* to the uses disclosed herein.

Accordingly, in a preferred embodiment the present invention relates to the use of a device of the present invention according to any of the embodiments disclosed herein for T cell expansion, wherein said use further comprises the use of a phorbol ester selected from PMA, 12-myristate-13-acetate, phorbol-12,13-didecanoate, phorbol-12,13-dibutyrate, and mezerin; a divalent ionophore selected from ionomycin or A23187; and optionally the protein transport inhibitor(s) brefeldin A and/or monensin. Even more preferably, the present invention relates to the use of a device of the present invention according to any of the embodiments disclosed herein for T cell expansion, wherein said use further comprises the use of the phorbol ester PMA, the ionophore ionomycin, and the protein transport inhibitors brefeldin A and monensin.

The uses described herein, may alternatively also be formulated as a use for T cell activation. Moreover, in other words the uses may also be formulated as a method for expanding/activating T cells using the device of the present invention. Accordingly, also corresponding aspects directed to a method for T cell expansion using the device of the present invention are provided in the present invention.

The present invention further relates to a T cell (e.g. a CD4+ T cell) obtainable or obtained by any of the methods for T cell expansion as described herein. Moreover, it also relates to a composition comprising a T cell (e.g. a CD4+ T cell) obtainable or obtained by any of the methods for T cell expansion described herein. Of course any of the embodiments, preferences and definitions described herein in the context of the methods for T cell expansion and the device of the present invention apply *mutatis mutandis.*

As illustrated in the appended Examples and Figures, by using the method and device for T cell expansion according to the present invention, a T cell population is obtained that differs from the population obtained using the commercially available Dynabeads and the protocol provided therewith in a static culture system and without external IL-2. The population of T cells (e.g. CD4+ T cells) obtainable or obtained with the method and device for T cell expansion according to present invention have a lower proportion of cells with a memory phenotype (CD45RO+ T cells) than the T cell population obtained with the Dynabeads method. As expected, this tendency is reverted for the naive phenotype (CD45RA+ T cells), although not yet statistically significant with the number of experiments performed. Such a different distribution of naive and memory phenotype is expected to be beneficial for the treatment of patients, e.g. T cells with such phenotypes are expected to have a longer in vivo survival and therefore be more efficient to treat diseases such as, for example, cancer.

Accordingly, the present invention provides for a composition enriched in T cells (e.g. CD4+ T cells) with a naive phenotype (CD45RA+ T cells) and/or depleted in T cells with a memory phenotype (CD45RO+ T cells). Enriched and/or depleted is in this context preferably determined by comparing the composition to a population of T cells resulting from the state-of-the-art Dynabeads method. Preferably, enriched means that at least (about) 36%, preferably at least (about) 38%, more preferably at least (about) 40%, even more preferably at least (about) 42%, and most preferably at least (about) 45% of the T cells in said composition have a naive phenotype, i.e. are CD45RA+ T cells. Depleted preferably means that at most (about) 86%, preferably at most (about) 85%, more preferably at most (about) 84,5%, and most preferably at most (about) 84,2 % of the T cells comprised in the composition have a memory phenotype, i.e. are CD45RO+ T cells. A composition may also be referred to as population herein.

The composition enriched in T cells (e.g. CD4+ T cells) with a naive phenotype (CD45RA+ T cells) and/or depleted in T cells with a memory phenotype (CD45RO+ T cells) is preferably a composition obtained or obtainable by a method for T cell expansion of the present invention, e.g. a method for T cell expansion using a device according to the present invention.

Methods for determining the proportion of T cells with a naive phenotype (CD45RA+ T cells) and/or the proportion of T cells with a memory phenotype (CD45RO+ T cells) are known in the art. For instance, the proportion may be determined by standard flow cytometry techniques. In these flow cytometry analyses for instance anti-human CD45RA FITC, CD45RO FITC, and iso CD45RA lgG2a FITC (Immunotools GmbH, Germany) may be used (see Reference [18]). When a composition does not exclusively comprise T cells, the proportion of T cells with a naive phenotype (CD45RA+ T cells) and/or the proportion of T cells with a memory phenotype (CD45RO+ T cells) may further involve determining CD3 expression as a T cell specific marker. Similarly, when a specific T cell subset is needed, e.g. CD4+ T cells, and the composition does not exclusively comprise it, the proportion of T cells with a naive phenotype (CD45RA+ T cells) and/or the proportion of T cells with a memory phenotype (CD45RO+ T cells) may further involve determining subset specific markers, e.g. CD4, in addition to the CD3 generic T cell marker.

Any of the T cells and compositions provided by the present invention are suitable for use in medicine (i.e. for use as a medicament). Accordingly, the present invention also relates to the respective T cells and/or compositions for use in medicine/for use as a medicament.

It is particularly envisaged that the T cells and/or compositions of the present invention may be employed in the treatment of cancer, a viral infection, or of an autoimmune disease. Thus, in one aspect the T cell and/or composition of the present invention may be a T cell/composition for use in the treatment of cancer, of a viral infection, or of an autoimmune disease. Similarly the composition of the present invention may be a composition for use in the treatment of cancer, of a viral infection, or of an autoimmune disease. A viral infection is preferably a chronic viral infection such as, for example, the human immunodeficiency virus (HIV). A preferred autoimmune disease in the context of the present invention is type 1 diabetes.

The present invention also relates to the following items:
1. A method for activation and/or expansion of T cells, the method comprising the steps
   (i) culturing a T cell in the presence of a phorbol ester, a divalent ionophore, and optionally one or more protein transport inhibitors; and
   (ii) culturing the T cell in the presence of an agent capable of activating CD3 and/or an agent capable of activating CD28.
2. The method of item 1, wherein said phorbol ester is phorbol-12-myristate-13-acetate (PMA).
3. The method of item 1 or 2, wherein said divalent ionophore is a Ca²⁺ ionophore, preferably selected from the group selected from: ionomycin and A23187.
4. The method of any one of items 1 to 3, wherein said one or more protein transport inhibitors are brefeldin A and/or monensin, preferably brefeldin A and monensin.
5. The method of any one of items 1 to 4, wherein said step (ii) is culturing the T cell in the presence of an agent capable of activating CD3 and an agent capable of activating CD28.
6. The method of any one of items 1 to 5, wherein said agent capable of activating CD3 is an anti-CD3 antibody.
7. The method of any one of items 1 to 6, wherein said agent capable of activating CD28 is an anti-CD28 antibody.
8. The method of any one of items 1 to 7, wherein the agent capable of activating CD3 is attached to a surface.
9. The method of any one of items 1 to 8, wherein the agent capable of activating CD28 is attached to a surface.
10. The method of any one of items 1 to 8, wherein the agent capable of activating CD28 is not attached to a surface.
11. The method of any one of items 1 to 8, wherein the agent capable of activating CD3 is attached to a surface and wherein the agent capable of activating CD28 is attached to a surface.
12. The method of any one of items 8 to 11, wherein said surface is a nanostructured surface of a device/member.
13. The method of item 12, wherein said nanostructured surface comprises TiO₂.
14. The method of item 12 or 13, wherein said nanostructured surface comprises quasi-hexagonally ordered nanoparticles (NPs).
15. The method of item 14, wherein said quasi-hexagonally ordered NPs are quasi-hexagonally ordered gold nanoparticles (AuNPs).
16. The method of item 14 or 15, wherein the distance between said NPs (interparticle distance) is from 35 nm to 150 nm.
17. The method of item 16, wherein said interparticle distance is about 35 nm.
18. The method of any one of items 8 to 17, wherein an RGD peptide is attached to the surface.
19. The method of item 18, wherein the surface on which the RGD peptide is attached is the area between said NPs (interparticle area).
20. The method of item 18 or 19, wherein said RGD peptide is a cyclic RGD peptide.
21. The method of any one of items 18 to 20, wherein said RGD peptide comprises a phosphonic acid.
22. The method of any one of items 1 to 21, wherein said T cell is a CD4⁺ T cell.
23. A device, wherein an RGD peptide is attached to the surface of the device.
24. The device of item 23, wherein said device is a device with a nanostructured surface (i.e. a nanoarray).
25. The device of item 23 or 24, wherein said surface, preferably the nanostructured surface of the device comprises TiO₂.
26. The device of any one of items 23 to 25, wherein said surface, preferably the nanostructured surface comprises quasi-hexagonally ordered NPs.
27. The device of item 26, wherein said quasi-hexagonally ordered NPs are quasi-hexagonally ordered AuNPs.
28. The device of item 26 or 27, wherein the distance between said NPs (interparticle distance) is from 35 nm to 150 nm.
29. The device of item 28, wherein said interparticle distance is about 35 nm.
30. The device of any one of items 23 to 29, wherein the surface to which RGD is attached is the area between said NPs (interparticle area).
31. The device of any one of items 23 to 30, wherein said RGD is a cyclic RGD.
32. The device of any one of items 23 to 31, wherein said RGD comprises a phosphonic acid.
33. The device of any one of items 23 to 32, wherein said device further comprises an agent capable of activating CD3 and/or an agent capable of activating CD28.
34. The device of any one of items 23 to 32, wherein said device further comprises an agent capable of activating CD3 and an agent capable of activating CD28.
35. The device of item 33 or 34, wherein said agent capable of activating CD3 is an anti-CD3 antibody.
36. The device of any one of item 33 or 34, wherein said agent capable of activating CD28 is an anti-CD28 antibody.
37. The device of any one of items 33 to 35, wherein the agent capable of activating CD3 is attached to the surface of the device.
38. The device of any one of items 33, 34, or 36, wherein the agent capable of activating CD28 is attached to the surface of the device.
39. The device of any one of items 33, 34 or 36, wherein the agent capable of activating CD28 is not attached to the surface of the device.
40. The device of any one of items 33 to 36, wherein the agent capable of activating CD3 is attached to the surface of the device and wherein the agent capable of activating CD28 is attached to the surface of the device.
41. Method for producing the device as defined in any one of items 23 to 40.
42. Use of the device as defined in any one of items 23 to 40 for activating and/or expanding T cells.
43. The use of item 42, further comprising the use of phorbol ester, a divalent ionophore, and optionally one or more protein transport inhibitors; or the device of any one of items 23 to 40, further comprising phorbol ester, a divalent ionophore, and protein transport inhibitors.
44. A T cell obtained or obtainable by the method of any one of items 1 to 22 or a composition comprising a T cell obtained or obtainable by the method of any one of items 1 to 22.
45. A composition enriched in T cells with a naive phenotype (CD45RA+) and/or depleted in T cells with a memory phenotype (CD45RO+).
46. The composition of item 45 obtained or obtainable by the method of any one of items 1 to 22.
47. The T cell of item 44 for use in medicine or the composition of any one of items 44 to 46 for use in medicine.
48. The T cell of item 44 for use in adoptive T cell therapy, preferably for use in the treatment of cancer, of a viral infection, or of an autoimmune disease; or the composition of any one of items 44 to 46 for use in adoptive T cell therapy, preferably for the use in the treatment of cancer, of a viral infection, or of an autoimmune disease.
49. The T cell for use of item 48 or the composition for the use of item 48, wherein said viral infection is a chronic viral infection.
50. The T cell for use of item 48 or the composition for the use of item 48, wherein said chronic viral infection is the human immunodeficiency virus (HIV).
51. The T cell for use of item 48 or the composition for the use of item 48, wherein said autoimmune disease is type 1 diabetes.

Antibodies or immunoglobulins are gamma globulin proteins consisting in their natural form of two large heavy chains and two small light chains linked by disulfide bonds. There are five types of mammalian Ig heavy chain: α, δ, ε, γ, and µ. The type of heavy chain present defines the class (isotype) of the antibody; these are IgA, IgD, IgE, IgG, and IgM antibodies, respectively. Each heavy chain has two regions, the constant region and the variable region. The constant region is nearly identical in all naturally occurring antibodies of the same isotype of the same species. A light chain also consists of one constant domain and one variable domain. In mammals there are two types of immunoglobulin light chains, lambda (λ) and kappa (κ). Although the general structure of all antibodies is very similar, the unique property of a given antibody is determined by the variable (V) regions. More specifically, variable loops, three on the light (V_{L}) and three on the heavy (V_{H)} chains, are responsible for binding to the antigen, i.e. for its antigen specificity. These loops are referred to as the Complementarity Determining Regions (CDRs). Because CDRs from both V_{H} and V_{L} domains contribute to the antigen-binding site, it is the combination of the heavy and the light chains, and not either alone, that determines the final antigen specificity.

The term "antibody", as used herein, means any polypeptide which is capable of binding to an antigen, wherein the binding specificity is determined by the CDRs. Hence, "antibody" is intended to relate to any polypeptide which comprises of at least one antigen binding fragment. Antigen binding fragments consist of at least the variable domain of the heavy chain and the variable domain of the light chain, arranged in a manner that both domains together are able to bind to the specific antigen. An "antibody" includes a complete antibody, or antibody fragments, e.g. Fab-, F(ab)₂-, or scFv-fragments.

With regard to the term "complete antibody", any antibody is meant to have a typical overall domain structure of a naturally occurring antibody (i.e. comprising a heavy chain of three or four constant domains and a light chain of one constant domain as well as the respective variable domains), even though each domain may comprise of further modifications, such as mutations, deletions, or insertions, which do not change the overall domain structure.

An "antibody fragment" also contains at least one antigen binding fragment as defined above, and exhibits the same function and specificity as the complete antibody from which the fragment is derived. Fab fragments may be generated by using the enzyme papain to cleave an immunoglobulin. The enzyme pepsin cleaves below the hinge region and, thus, below the disulfide bonds, so that an F(ab)₂ fragment is formed. Moreover, the variable regions of the heavy and light chains can be fused together to form a single chain variable fragment (scFv).

In addition, the term "antibody" is intended to comprise all above-mentioned immunoglobulin isotypes, i.e. the antibody may be an IgA, IgD, IgE, IgG, or IgM antibody, including any subclass of these isotypes. Preferably, the antibody is an IgG antibody, more preferably an IgG1 or IgG2 antibody. Since the antibody may be expressed and produced recombinantly, the antibody may also comprise two different constant regions of heavy chains, e.g. one IgG1 and one IgG2 heavy chain, or heavy chains from different species. However, the heavy chains are preferably from the same species. Moreover, the antibody may comprise either a lambda or a kappa light chain.

In context of the present invention, the term "nanostructured surface" refers to a surface of a support/member/device (also referred to as base structure or substrate) that is decorated with an array of NPs, preferably a quasi-hexagonal array of NPs, most preferably a quasi-hexagonal array of AuNPs.

A device having a nanostructured surface (e.g. upper surface) according to the present invention may also be referred to as "nanoarray" or "nanostructured substrate" in the context of the present invention. These terms have been used interchangeably in the literature.

In context of the present invention, the term "nanoparticle" means particles between 1 and 100 nanometers (nm) in size. The particles may have different shapes. Preferred shapes are spheres of diameters between 3 and 10 nm. Preferably NPs consist of an inorganic material, preferably gold or silver, most preferably gold.

In context of the present invention, the term "quasi-hexagonally ordered" means that the order parameter of the structure formed by the NPs is at least 0.5. The order parameter of the nanostructured surfaces and the interparticle distances are calculated with a software that assumes a six-closest-neighbor model (see Reference [10]) and gives a parameter from 0 to 1 to quantify the order, being 1 a perfectly hexagonal structure and 0 a completely random distribution.

As used herein, the terms "comprising" and "including" or grammatical variants thereof are to be taken as specifying the stated features, integers, steps, or components but do not preclude the addition of one or more additional features, integers, steps, components, or groups thereof. This term encompasses the terms "consisting of" and "consisting essentially of." Thus, the terms "comprising"/"including"/"having" mean that any further component (or likewise features, integers, steps, and the like) can be present.

The term "consisting of' means that no further component (or likewise features, integers, steps, and the like) can be present.

The term "consisting essentially of" or grammatical variants thereof when used herein are to be taken as specifying the stated features, integers, steps, or components but do not preclude the addition of one or more additional features, integers, steps, components, or groups thereof but only if the additional features, integers, steps, components, or groups thereof do not materially alter the basic and novel characteristics of the claimed composition, device, or method. Thus, the term "consisting essentially of' means that specific further components (or likewise features, integers, steps, and the like) can be present, namely those not materially affecting the essential characteristics of the composition, device, or method. In other words, the term "consisting essentially of" (which can be interchangeably used herein with the term "comprising substantially"), allows the presence of other components in the composition, device, or method in addition to the mandatory components (or likewise features, integers, steps, and the like), provided that the essential characteristics of the device or method are not materially affected by the presence of other components.

The term "method" refers to manners, means, techniques, and procedures for accomplishing a given task including, but not limited to, those manners, means, techniques, and procedures either known to, or readily developed from known manners, means, techniques, and procedures by practitioners of the chemical, biological, and biophysical arts.

As used herein, the term "isolated" refers to a composition that has been removed from its *in vivo* location. Preferably the isolated compositions or compounds of the present invention are substantially free from other substances (e.g., other proteins or other compounds) that are present in their *in vivo* location (i.e. purified or semi-purified compositions or compounds.)

As used herein the term "about" refers to ± 10%.

The present invention is additionally described by means of the following illustrative non-limiting Figures and Examples that provide a better understanding of the present invention and of its many advantages.

The Figures show:
**Figure 1****.**

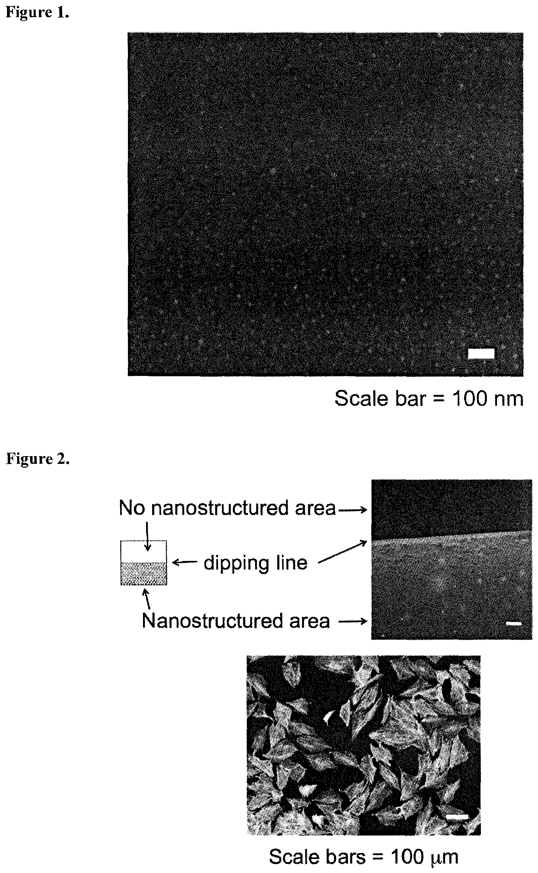

   Figure 1 shows a scanning electron microscopy (SEM) image of a TiO₂ surface (dark grey background) decorated with an array of AuNPs (white dots) separated ca. 35 nm.
**Figure 2****.**
   On the top-right, a fluorescence image of a surface is provided that was only half decorated with AuNPs by dip-coating, so that a border between nanostructured and non-nanostructured areas was obtained (see scheme on the top-left). By functionalizing the whole surface with a fluorescently marked anti-CD3, fluorescence was only obtained on the nanostructured area. On the bottom, a fluorescence image of the actin filaments and focal adhesions of rat embryonic fibroblast (REF) cells that were seeded on a functionalized nanostructured surface is provided. They adhered everywhere due to the functionalization of TiO₂ with RGD as it can be seen by their elongated shape, the distribution of actin filaments, and the presence of focal adhesions.
**Figure 3****.**

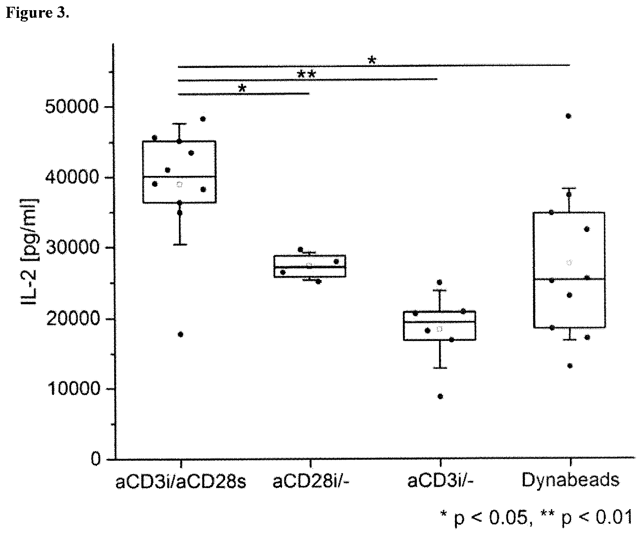

   Figure 3 shows the IL-2 secretion of prestimulated CD4+ T cells with PMA/ionomycin (plus protein transport inhibitors) using different activation/expansion methods:
   1) Soluble anti-CD28 and nanostructured surfaces consisting of an RGD-functionalized TiO₂ surface decorated with attached anti-CD3 on the quasi-hexagonal array of AuNPs separated ca. 35 nm (aCD3i/aCD28s);
   2) Nanostructured surfaces consisting of an RGD-functionalized TiO₂ surface decorated with anti-CD28 attached to the quasi-hexagonal array of AuNPs separated ca. 35 nm (aCD28i/-);
   3) Nanostructured surfaces consisting of an RGD-functionalized TiO₂ surface decorated with anti-CD3 attached to the quasi-hexagonal array of AuNPs separated ca. 35 nm (aCD3i/-);
   4) Dynabeads method: positive control.
**Figure 4****.**

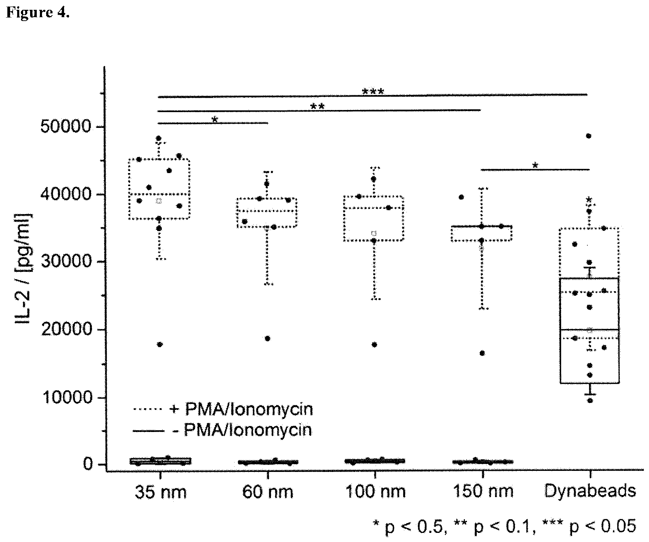

   Figure 4 shows the IL-2 secretion of prestimulated (with PMA/ionomycin (plus protein transport inhibitors)) CD4+ T cells obtained using soluble anti-CD28 and nanostructured surfaces consisting of an RGD-functionalized TiO₂ surface decorated with attached anti-CD3 on quasi-hexagonal arrays of AuNPs separated x nm (x= 35 nm; 60 nm; 100 nm; 150 nm). The positive control "Dynabeads" is also included.
**Figure 5****.**

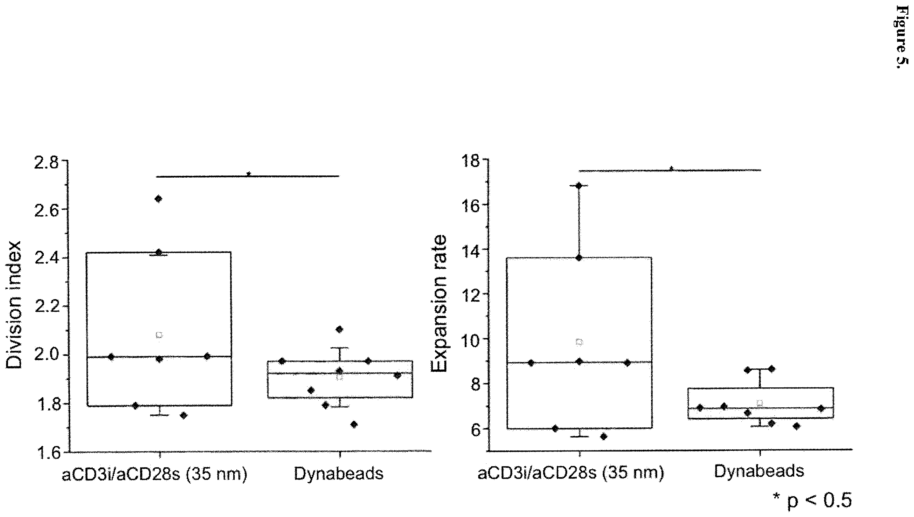

   Figure 5 shows the proliferation rates of prestimulated CD4+ T cells on day 7. The expansion rate and division index are higher for cells expanded on the aCD3i/aCD28s system (with interparticle distances of ca. 35 nm) than for parallel experiments using the state-of-the-art Dynabeads method.
**Figure 6****.**

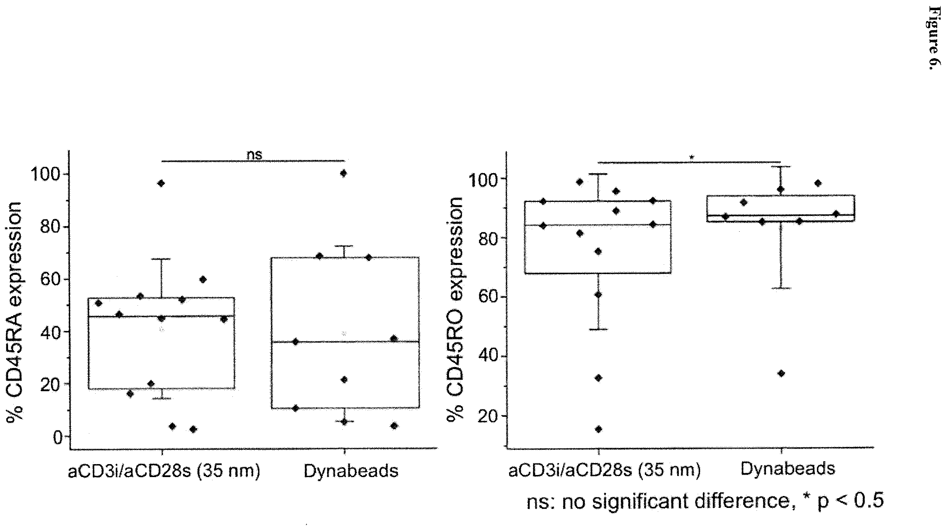

   Figure 6 shows the percentages of naive (CD45RA+) and memory (CD45RO+) phenotypes of prestimulated CD4+ T cells on day 6. The CD45RO+ expression is lower in the aCD3i/aCD28s system (interparticle distance of ca. 35 nm) than for parallel experiments using the Dynabeads method, whereas the CD45RA+ expression is higher.

The following Example illustrates the invention.

### Example 1: A method and a device to increase primary human CD4+ T cell ex vivo expansion by using adhesive nanostructured surfaces and costimulatory signals

With the objective of obtaining large amounts of CD4+ T cells in the laboratory, the following protocol was developed.

### Production of nanostructured surfaces on glass supports

In the present Example, glass slides were sputtered with a layer of 200 nm TiO₂ to provide a glass slide having a surface that consists of TiO₂. Although not employed in the present Example, in principle also other supports than glass such as silicon dioxide or silicon wafers, other coating strategies than sputtering such as evaporation, and other surface widths than 200 nm can be used provided that the surface consists of TiO₂. The size of the support can be varied according to technical requirements. In the present Example, commercial glass slides (squares of 24 mm x 24 mm) were washed 1 h in a mixture of hydrogen peroxide and sulfuric acid (1:3). The surfaces were rinsed with milliQ water prior to sputtering. Afterwards, the sputtered surfaces were washed in an ultrasonic bath with cycles of 10 min using *n*-heptane, isopropanol, milliQ water, and acetone. Finally, they were plasma etched with oxygen at 150 W and 0.4 mbar for 1 min.

Quasi-hexagonally ordered AuNPs were deposited on the above mentioned surfaces by BCML following a protocol previously described in References [19]. In the present experiment, block copolymers consisting of polystyrene (PS) and 2-polyvinylpyridine (P2VP), PS(x)-*b*-P2VP(y) where x and y varied between 100 and 2000 and PS(x) > P2VP(y), were used. As described in the cited literature (see References [19]), other polymer compositions can provide equivalent results, e.g. PS(x)-*b*-P4VP(y) where P4VP is 4-polyvinylpyridine. The specific polymer, which was chosen according to the interparticle distance needed (see Table 1), was stirred in toluene for 24 h. During this period, micelles consisting of a hydrophobic PS shell and a hydrophilic P2VP core are formed. Although these micelles can be loaded with various metal salts and inorganic metal complexes (see References [19]), this present Example has been focused on gold (III) chloride trihydrate. This compound was added into the micellar solutions and stirred for 24 h. The salt dissolves into the polar micellar cores, where it loses a proton that binds to the nitrogen atom of the P2VP chain. The electrostatic interactions created between the negatively charged AuCl₄⁻ and the positively charged NH⁺ of the polymer chains stabilize the micelles. The size of the resulting AuNPs could be varied by changing the loading (L) of the micelles (L = units (HAuCl4) / units (P2VP)). Table 1 below shows the specific parameters used to prepare the solutions used for the experiments described in the present Example.

**Table 1**

| Solution | Polymer | Polymer concentration [mg/ml] | Loading (L) | Interparticle distance [nm] | Standard deviation [nm] |
|---|---|---|---|---|---|
| 1 | PS(240)-P2VP(143) | 5 | 0,5 | 35 | 7 |
| 2 | PS(1056)-P2VP(671) | 5 | 0,35 | 60 | 11 |
| 3 | PS(1056)-P2VP(671) | 4 | 0,25 | 100 | 12 |
| 4 | PS(5438)-P2VP(713) | 1 | 0,25 | 150 | 40 |

To obtain the nanostructured surfaces, two coating methods were employed, dip- and spin-coating. The first one was employed to prepare test surfaces used to optimize the functionalization protocol, as explained below. All surfaces used for cell experiments were obtained by spin-coating. In the dip-coating method, surfaces are immersed at a constant speed into a chosen micellar solution and pulled out. Due to capillary forces during the evaporation of the solvent, a compact two-dimensional quasi-hexagonally structured monolayer of micelles loaded with gold (III) ions is obtained on the TiO₂ surface. The distance between AuNPs can be finely adjusted by changing the velocity of the dip-coating process (increasing the speed results in smaller distances). In this Example, a velocity of 24 mm/min was used. In the spin-coating method, a specific volume of micellar solution is deposited onto the surface, which is rotating at a high speed to thin the fluid and dry the excess of solvent from the resulting film. The acceleration of the rotating support, the final speed (increasing the speed results in larger distances), and the rotation time can be set on the spin coater instrument to tune the interparticle distance. In our preferred configuration (used for the T cell experiments), 25 µl of micellar solution were poured onto 24 mm x 24 mm surfaces using the parameters described in Table 2.

**Table 2**

| Solution | Polymer | Spinning velocity [rpm] | Time [min] | Interparticle distance [nm] | Standard deviation [nm] |
|---|---|---|---|---|---|
| 1 | PS(240)-P2VP(143) | 6000 | 1 | 35 | 7 |
| 2 | PS(1056)-P2VP(671) | 3000 | 1 | 60 | 11 |
| 3 | PS(1056)-P2VP(671) | 10000 | 1 | 100 | 12 |
| 4 | PS(5438)-P2VP(713) | 8000 | 1 | 150 | 40 |

In the next step, the nanostructured surfaces were treated with an oxygen plasma at 120 W and 0,4 mbar for 10 min. Thus, gold (III) ions were reduced to metallic gold, the block copolymer was destroyed, and the nanostructured surfaces were formed (Figure 1). Finally, the surfaces were further treated in an oven at a temperature of 500 °C for 48 h to reinforce the anchoring of the AuNPs to the surface and avoid the removal of particles by cells.

### Functionalization of nanostructured surfaces

Once the nanostructured surfaces were produced, they were functionalized with a cell-adhesive RGD background on the TiO₂ surface and anti-CD3 antibody (anti-CD3) or anti-CD28 antibody (anti-CD28) on the AuNPs.

The antibody functionalization on the AuNPs was based on a protocol previously reported (see Reference [6]c). The differences were that 1) the concentration of the thiolated molecule was reduced to 0.5 mM and the incubation time to 60 min, 2) the incubation of the NiCl₂ solution and protein G was done in two steps of 15 and 60 min, respectively, and 3) HBS buffer was used instead of a PBS buffer during the steps.

Lab-Tek chambers (8 wells, ThermoFisher Scientific, US) were employed for the cell experiments. However, in principle other chamber slide systems and/or equivalent products as well as chambers of other sizes and shapes can be used. In the present Example, the bottom of the chambers were the glass slides sputtered with a layer of 200 nm TiO₂ that were decorated with quasi-hexagonal arrays of AuNPs. Such surfaces were glued to the chambers with a biocompatible adhesive. Once dried after gluing, the nanostructured surfaces were sterilized with an aqueous solution of 50% ethanol for 30 min. In principle, other sterilization protocols such as UV irradiation could also be used.

For functionalizing the interparticle areas (i.e. the areas between the NPs) with the RGD peptide, a cyclic RGD was used. This functionalization was performed to enhance the cell-surface interaction and to prevent unspecific protein adsorption to TiO₂. Specifically, nanostructured surfaces were incubated with a 25 µM aqueous solution of a cyclic RGD molecule that contains phosphonic acids as anchor groups (see "peptide 1:RGD-spacer-tetraphosphonate" in Reference [14], which is herewith incorporated in its entirety) for 12 h at RT. The employed peptide had the following formula:

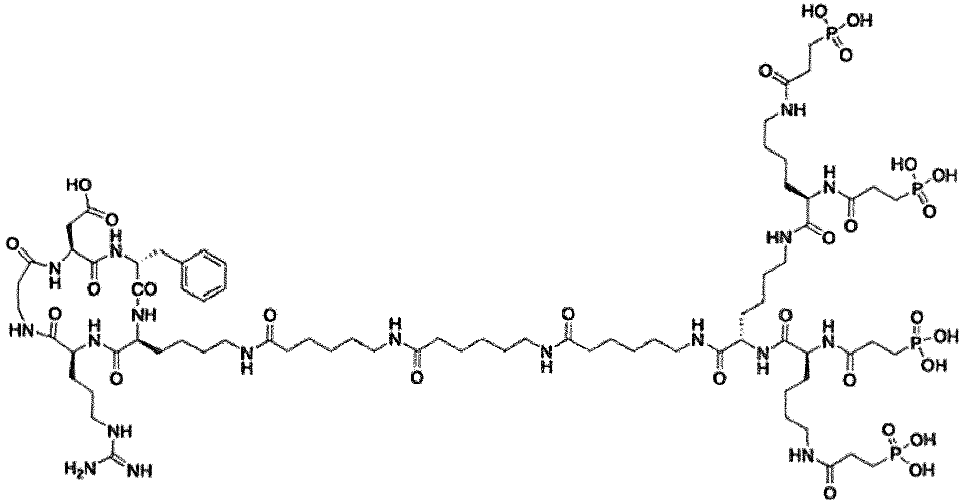

The RGD peptide motif has been identified as a minimal fragment of the ECM protein fibronectin that may stimulate cell adhesion through integrin binding (see Reference [13]).

However, the RGD peptide has not been employed in *ex vivo* T cell expansion and in particular not in T cell expansion with an anti-CD3 antibody and an anti-CD28 antibody. The employed cyclic RGD binds strongly to TiO₂ via their phosphonic acid anchor groups. Using the binding of phosphonic acids to TiO₂ in order to immobilize RGD has the advantage that this immobilization remains stable under aqueous conditions (e.g. during subsequent culturing of T cells in presence of medium).

To subsequently functionalize the AuNPs with the indicated antibodies, surfaces were washed with deionized water and subsequently incubated in a 0.5 mM solution of a thiolated nitrilotriacetic acid (HS-(CH₂)₁₁-(EG)₃-NTA) molecule (Prochimia, Poland) dissolved in an aqueous solution of 50 % ethanol for 1 h. NTA is a chelating agent and in aqueous solutions forms stable complexes with metal ions such as Ni²⁺. Subsequently, the nanostructured surfaces were washed with water and equilibrated with an HBS solution for 10 min. Afterwards, they were immersed in a 10 mM NiCl₂ solution for 30 min, followed by a 10 µg/ml solution of a His6-protein G (BioCat GmbH, Germany) during 1 h. Protein G was coupled to the NTA by means of a coordination complex with Ni²⁺ through its histidine tag (His6). Although not used in the present Example, the histidine tag could of course also include a higher number of histidine groups. Moreover, protein G is an immunoglobulin-binding protein that binds the Fc region of antibodies ensuring their site-directed immobilization and therefore enabling maximal biological activity. With this objective, surfaces were incubated in a 10 µg/ml solution of an anti-CD3 (or the fluorescently labeled Alexa 488 anti-CD3 or anti-CD28) for 1 h. Finally, the nanostructured surfaces were extensively washed with HBS for 6 h at 4 °C under mild shaking. If not stated otherwise, washing steps with HBS were performed after each functionalization step under atmospheric conditions. The volumes used for the incubation and washing steps were 150 µl and 400 µl, respectively.

### Analysis of the performance of the functionalization protocol

To analyze the performance of the described protocol, different surfaces were decorated with AuNPs on only one half of the surface by dip-coating (see Figure 2, scheme). Thus, a border between nanostructured and non-nanostructured areas was obtained. By functionalizing the whole surface with a fluorescently marked anti-CD3 antibody (labeled with Alexa 488; eBioscience GmbH, Germany), fluorescence was only observed on the nanostructured area, confirming that the antibody was successfully immobilized on the AuNPs (Figure 2, top).

Moreover, the low fluorescence of the area above the dipping line indicates that unspecific adsorption of the antibodies was minimal.

### Analysis of the cell-adhesive properties of the RGD-functionalized TiO₂ surface

To evaluate the cell-adhesive properties of the RGD-functionalized TiO₂, REF cells were seeded on the functionalized nanostructured surfaces. For that, REF cells were suspended in Dulbecco's modified Eagle's medium (DMEM) with 10 % FBS and 1 % P/S. Then, they were seeded at a density of 5000 cells/cm², and incubated at 37 °C and 5 % CO2 for 12 h. Afterwards, cells were fixed with 4 % of paraformaldehyde (PFA, Serva, Germany) for 10 min and permeabilized with 0,1 % Triton X-100 (Sigma Aldrich, US) for 3 min. Then, a blocking step with 4 % bovine serum albumin (BSA, Serva, Germany) for 30 min was performed. Cells were then incubated first with a monoclonal anti-vinculin antibody produced in mouse (1:400; Sigma Aldrich, US) for 1 h and then with 4',6-diamidino-2-phenylindole (DAPI) (1:1000; Serva, Germany), donkey anti-mouse Alexa Fluor 568 (1:300; Abcam, UK), and Alexa Fluor 488 phalloidin (1:50; Thermo Fisher Scientific, US) for 30 min in the dark. All solutions and washings between steps were performed in PBS. Finally, surfaces were mounted in ProLong Gold Antifade Reagent (Termo Fisher Scientific, US) and analyzed by optical and fluorescence microscopy with a Zeiss Axiovert 200M microscope (Carl Zeiss AG, Germany) as shown in Figure 2 (bottom). Cells spread on the surfaces and actin filaments and focal adhesions were observed, confirming the cell-adhesive properties provided by RGD.

### T cell experiments

Primary human CD4+ T cells were purified from blood of healthy adult donors through a density gradient centrifugation to recuperate the PBMCs. Then, the needed amount of CD4+ T cells was obtained using a commercially available kit (CD4+ T cell isolation kit, Miltenyi Biotech, Germany), which is based on a magnetic purification. Briefly, all non-CD4+ T cells are marked with anti-biotin, which further reacts with magnetic beads that stay in the chromatography column placed on a magnet. Thus, only CD4+ T cells pass through the column. The isolated CD4+ T cells were then suspended in a standard T cell medium (RPMI Medium 1640 Glutamax with 10 % FBS and 1 % P/S) and used for experiments. In the experiments of the present Example, only cells that were at least 95 % positive for both CD3+ and CD4+ according to flow cytometry were used. In some of the experiments (as indicated in the Figures and the corresponding Figure legends), a prestimulatory step consisting of incubating the cells in the standard T cell medium supplemented with a 2 µl/ml costimulatory cocktail was performed for 5 h following the recommendations of the supplier (eBioscience GmbH, Germany). Specifically, this costimulatory cocktail contained PMA (40,5 µM), ionomycin (670 µM), brefeldin A (5,3 mM), and monensin (1 mM) in ethanol. The combination of PMA and ionomycin triggers IL-2 synthesis and the expression of its receptor through the activation of PKC and an increase of the cytosolic-free calcium concentration, whereas the protein transport inhibitors brefeldin A and monensin promote the accumulation of secreted proteins (see References [7]). In the next step, CD4+ T cells were seeded on the functionalized nanostructured surfaces (as indicated in the Figures and corresponding Figure legends) at a concentration of 10⁶ cells/ml for activation analysis. Where indicated in the Figures and/or Figure legends soluble anti-CD28 (2 µl/ml; also referred to as aCD28s) was added in selected samples due to its potential role as costimulatory agent through its binding to the receptor CD28 on the T cell surface (see References [4]). A beads-based method (Dynabeads method) for T cell activation and proliferation was used as positive control in some of the experiments. Dynabeads were used at a concentration of 25 µl bead suspension/ml as recommended by the supplier (article Nr. 11131D; Termo Fisher Scientific, US). More specifically, in this Example, beads and cells were mixed in a static culture system and no external IL-2 was added, as such cytokine was used as activation marker. The Dynabeads method is considered the state-of-the-art commercial expansion method and is used in many clinical trials. In our preferred experiments, the exact volumes used are summarized in Table 3.

**Table 3.**

| | Cell suspension (2·10⁶ cells/ml) | Medium | aCD28 | Dynabeads |
|---|---|---|---|---|
| Samples (35, 60, 100, 150 nm) | 187,5 | 186,75 | 0,75 | 0 |
| 35 nm without aCD28 | 187,5 | 187,5 | 0 | 0 |
| Positive control (Dynabeads) | 187,5 | 178,12 | 0 | 9,38 |
| Negative control (Dish) | 187,5 | 187,5 | 0 | 0 |

### Analysis of IL-2 secretion as early activation marker by T cells

16 to 20 h after CD4+ T cell seeding, IL-2 secretion was analyzed through an ELISA to evaluate T cell activation (human IL-2 Quantikine ELISA kit; R&D Systems, US). Figure 3 shows the IL-2 secretion of prestimulated CD4+ T cells using different activation methods: 1) Soluble anti-CD28 and nanostructured surfaces consisting of an RGD-functionalized TiO₂ surface decorated with anti-CD3 immobilized on a quasi-hexagonal array of AuNPs separated ca. 35 nm (aCD3i/aCD28s); 2) Nanostructured surfaces consisting of an RGD-functionalized TiO₂ surface decorated with anti-CD28 immobilized on a quasi-hexagonal array of AuNPs separated ca. 35 nm (aCD28i/-); 3) Nanostructured surfaces consisting of an RGD-functionalized TiO₂ surface decorated with anti-CD3 immobilized on a quasi-hexagonal array of AuNPs separated ca. 35 nm (aCD3i/-); 4) Dynabeads method: positive control. The IL-2 secretion of CD4+ T cells stimulated on RGD-functionalized TiO₂ nanostructured surfaces with immobilized anti-CD3 and soluble anti-CD28 (aCD3i/aCD28s) was significantly higher than the obtained in any other configuration. Specifically, the IL-2 secretion was higher than the ones obtained by methods that only contained one antibody (aCD28i/- or aCD3i/-). Interestingly, such IL-2 secretion was also significantly higher than the one obtained with the state-of-the-art commercial Dynabeads. It is also worth mentioning that all the antibody-containing activation strategies used resulted in higher IL-2 secretion than cells seeded on dishes, which were only prestimulated with PMA/ionomycin and protein transport inhibitors. Furthermore, the IL-2 secretions of prestimulated and untreated CD4+ T cells obtained using Dynabeads or soluble anti-CD28 and nanostructured surfaces consisting of an RGD-functionalized TiO₂ surface decorated with anti-CD3 immobilized on AuNPs separated x nm (x = 35; 60; 100; 150) were compared (see Figure 4). The absence of a prestimulatory step with PMA/ionomycin and the protein transport inhibitors results in considerably less IL-2 secretion when using functionalized nanostructured surfaces (median < 1 ng / ml). In this situation, the Dynabeads method is the most efficient activation system. Nevertheless, prestimulated CD4+ T cells surprisingly showed the highest IL-2 secretion on nanostructured surfaces. Moreover, cells seeded on the surfaces with the largest interparticle distance (150 nm) still show surprisingly higher IL-2 concentrations (median > 30 ng / ml) than T cells cultured with Dynabeads.

### Analysis of T cell proliferation

Proliferation rates of T cells obtained 7 days after seeding using the preferred system consisting of prestimulated CD4+ T cells seeded on RGD/anti-CD3-functionalized nanostructures with interparticle distances of about 35 nm costimulated with soluble anti-CD28 were analyzed. For analyzing the proliferation rates, CD4+ T cells were stained with carboxyfluorescein succinimidyl ester (CFSE) prior to seeding and subsequently analyzed by flow cytometry. For these experiments, only 0.5x10⁶ cells/ml were seeded to avoid saturation while keeping constant the proportions of anti-CD28 and Dynabeads described in Table 3. Figure 5 shows that the expansion rate and division index are higher for cells expanded on functionalized nanostructured surfaces than for cells expanded on Dynabeads.

### Phenotype analysis of expanded T cells

Percentages of naive (CD45RA+) and memory (CD45RO+) phenotypes of the expanded samples were analyzed on day 6 by flow cytometry due to its influence on T cell function. Antihuman CD45RA FITC, CD45RO FITC, and iso CD45RA lgG2a FITC (Immunotools GmbH, Germany) were used. CD4+ T cells expanded on RGD/anti-CD3-functionalized nanostructures with interparticle distances of ca. 35 nm costimulated with soluble anti-CD28 and prestimulated with PMA/ionomycin and protein transport inhibitors produced less cells with a memory phenotype than Dynabeads. Not surprisingly, this tendency was reverted for the naive phenotype (CD45RA+ T cells), although more experiments would be required to achieve statistical confirmation. Such a different distribution of naive and memory phenotype is expected to be beneficial for the treatment of patients, e.g. T cells with such phenotypes are expected to have a longer *in vivo* survival and therefore be more efficient to treat e.g. cancer.

Throughout the application text several references have been cited. All references cited herein are fully incorporated by reference. Having now fully described the invention, it will be understood by a person skilled in the art that the invention may be practiced within a wide and equivalent range of conditions, parameters, and the like, without affecting the spirit or scope of the invention or any embodiment thereof.

References cited herein have either been indicated directly in the application text or have been cited by indicating reference numbers. The respective references corresponding to the reference numbers used are listed in the following:

### References cited herein

**References [1]:**
   **a)** M. Kalos, B. L. Levine, D. L. Porter, S. Katz, S. A. Grupp, A. Bagg, C. H. June, Sci. Transl. Med. 2011, 3, 1;
   **b)** D. L. Porter, B. L. Levine, M. Kalos, A. Bagg, C. H. June, N. Eng. J. Med. 2011, 365, 725;
   **c)** D. L. Porter, W. -T. Hwang, N. V. Frey, S. F. Lacey, P. A. Shaw, A. W. Loren, A. Bagg, K. T. Marcucci, A. Shen, V. Gonzalez, D. Ambrose, S. A. Grupp, A. Chew, Z. Zheng, M. C. Milone, B. L. Levine, J. J. Melenhorst, C. H. June, Sci. Transl. Med. 2015, 7, 303ra139.
**References [2]:**
   **a)** M. O. Butler, N. Hirano, Immunol. Rev. 2014, 257, 191;
   **b)** J. G. Crompton, M. Sukumar, N. P. Restifo, Immunol. Rev. 2014, 257, 264.
**Reference [3]:**
   D. Zehn, S. Y. Lee, M. J. Bevan, Nature 2009, 458, 211.
**References [4]:**
   **a)** C. H. June, J. A. Ledbetter, P. S. Linsley, C. B. Thompson, Immunol. Today 1990, 11, 211;
   **b)** L. Koulova, E. A. Clark, G. Shu, B. Dupont, J. Exp. Med. 1991, 173, 759;
   **c)** B. L. Levine, W. B. Bernstein, M. Connors, N. Craighead, T. Lindsten, C. B. Thompson, C. H. June, J. Immunol. 1997, 159, 5921.
**Reference [5]:**
   J. M. Curtsinger, C. S. Schmidt, A. Mondino, D. C. Lins, R. M. Kedl, M. K. Jenkins, M. F. Mescher, J. Immunol. 1999, 162, 3256.
**References [6]:**
   **a)** D. Delcassian, D. Depoil, D. Rudnicka, M. Liu, D. M. Davis, M. L. Dustin, I. E. Dunlop, Nano Lett. 2013, 2013, 5608;
   **b)** J. Deeg, M. Axmann, J. Matic, A. Liapis, D. Depoil, J. Afrose, S. Curado, M. L. Dustin, J. P. Spatz, Nano Lett. 2013, 13, 5619;
   **c)** J. Matic, J. Deeg, A. Scheffold, I. Goldstein, J. P. Spatz, Nano Lett. 2013, 13, 5090.
**References [7] :**
   **a)** Y. K. Yip, R. H. L. Pang, J. D. Oppenheim, M. S. Nachbar, D. Henriksen, I. Zerebeckyj-Eckhardt, J. Vilcek, Infect. Immun., 1981, 34, 131;
   **b)** A. Truneh, F. Albert, P. Golstein, A. -M. Schmitt-Verhulst, Nature 1985, 313, 318;
   **c)** T. Chatila, L. Silverman, R. Miller, R. Geha, J. Immunol. 1989, 143, 1283;
   **d)** N. Berry, Y. Nishizuka, Eur. J. Biochem. 1990, 189, 205;
   **e)** S. Hashimoto, Y. Takahashi, Y. Tomita, T. Hayama, S. Sawada, T. Horie, C. C. McCombs, J. P. Michalski, Scand. J. Immunol. 1991, 33, 393;
   **f)** H. R. B. Pelham, Cell 1991, 67, 449;
   **g)** R. D. Klausner, J. G. Donaldson, J. Lippincott-Schwartz, J. Cell BioL 1992, 116, 1071;
   **h)** H. H. Mollenhauer, D. J. Morré, L. D. Rowe, Biochim. Biophys. 1990, 1031, 225.
**Reference [8]:**
   A. Weiss, J. B. Imboden, Adv. Immunol. 1987, 41, 1.
**Reference [9]:**
   A. B. Lyons, C. R. Parish, J. Immunol. Methods 1994, 171, 131.
**Reference [10]:**
   A. R. Kansal, T. M. Truskett, S. Torquato, J. Chem. Phys. 2000, 113, 4844.
**References [11]:**
   **a)** E. Gomar-Nadal, J. P. Luis, D. B. Amabilino, Chem. Soc. Rev. 2008, 37, 490;
   **b)** D. S. Ginger, H. Zhang, C. A. Mirkin, Angew. Chem. Int. Ed. 2004, 43, 30;
   **c)** S. Y. Chou, P. R. Krauss, P. J. Renstrom, Science 1996, 272, 85.
**References [12]:**
   **a)** J. Guasch, B. Conings, S. Neubauer, F. Rechenmacher, K. Ende, C. G. Rolli, C. Kappel, V. Schaufler, A. Micoulet, H. Kessler, H. -G. Boyen, E. A. Cavalcanti-Adam, J. P. Spatz, Adv. Mater. 2015, 27, 3737;
   **b)** J. Guasch, J. Diemer, H. Riahinezhad, S. Neubauer, H. Kessler, J. P. Spatz, Chem. Mater. 2016, 28, 1806.
**Reference [13]:**
   M. D. Pierschbacher, E. Ruoslahti, Nature 1984, 309, 30.
**Reference [14] :**
   C. Mas-Moruno, P. M. Dorfner, F. Manzenrieder, S. Neubauer, U. Reuning, R. Burgkart, H. Kessler, J. Biomed. Mater. Res. Part A 2013, 101A, 87.
**Reference [15]:**
   S. J. Bogdanowich-Knipp, S. Chakrabarti, T. D. Williams, R. K. Dillman, T. J. Siahaan, J. Peptide Res. 1999, 53, 530.
**Reference [16]:**
   F. Rechenmacher, S. Neubauer, C. Mas-Moruno, P. M. Dorfner, J. Polleux, J. Guasch, B. Conings, H. -G. Boyen, A. Bochen, T. R. Sobahi, R. Burgkart, J. P. Spatz, R. Fässler, H. Kessler, Chem. Eur. J. 2013, 19, 9218.
**Reference [17]:**
   Y. J. Son, H. Kim, K. W. Leong, H. S. Yoo, ACS Nano 2014, 7, 9771.
**Reference [18]:**
   C. R. Mackay, Nature 1999, 401, 659.
**References [19]:**
   **a)** J. P. Spatz, S. Moessmer, C. Hartmann, M. Moeller, Langmuir 2000, 16, 407;
   **b)** T. Herzog, M. Möller, S. Mössmer, J. P. Spatz, P. Ziemann. Eur. Pat. Apl. 1027157, Aug. 6, 2003**;**
   **c)** T. Lohmueller, D. Aydin, M. Schwieder, C. Morhard, I. Louban, C. Pacholski, J. P. Spatz, Biointerphases 2011, 6, MR1;
   **d)** J. P. Spatz, T. Lohmueller, M. Arnold. U. S. Patent 8,257,798, Sep. 4, 2012**.**

## Claims

1. A method for expansion of T cells, the method comprising the steps
(i) culturing a T cell in the presence of a phorbol ester and a divalent ionophore; and
(ii) culturing the T cell in the presence of an agent capable of activating CD3 and/or an agent capable of activating CD28.

2. The method of claim 1, wherein said phorbol ester is phorbol-12-myristate-13-acetate (PMA).

3. The method of claim 1 or 2, wherein said divalent ionophore is ionomycin or A23187.

4. The method of any one of claims 1 to 3, wherein in the culturing in (i) further comprises the presence of one or more protein transport inhibitors, wherein said one or more protein transport inhibitors preferably comprise monensin and/or brefeldin A, and most preferably wherein said one or more protein transport inhibitors are monensin and brefeldin A.

5. The method of any one of claims 1 to 4, wherein said step (ii) is culturing the T cell in the presence of an agent capable of activating CD3 and an agent capable of activating CD28.

6. The method of any one of claims 1 to 5, wherein said agent capable of activating CD3 is an anti-CD3 antibody.

7. The method of any one of claims 1 to 6, wherein said agent capable of activating CD28 is an anti-CD28 antibody.

8. The method of any one of claims 1 to 7, wherein the agent capable of activating CD3 is attached to a surface.

9. The method of claim 8, wherein said surface is a nanostructured surface, preferably a nanostructured surface of a nanoarray.

10. The method of claim 8 or 9, wherein said surface comprises TiO₂ and quasi-hexagonally ordered nanoparticles (NPs), such as gold NPs (AuNPs).

11. The method of claim 10, wherein said agent capable of activating CD3 is exclusively attached to said NPs.

12. The method of claim 10 or 11, wherein the distance between said NPs (interparticle distance) is from 20 nm to 150 nm, preferably wherein the distance between said NPs (interparticle distance) is about 35 nm.

13. The method of any one of claims 8 to 12, wherein an RGD peptide is attached to said surface, preferably exclusively attached to the area between said NPs (interparticle area) of said surface.

14. The method of claim 13, wherein said RGD peptide is a cyclic RGD peptide and/or wherein said RGD peptide comprises a phosphonic acid.

15. The method of any one of claims 1 to 14, wherein said T cell is a CD4+ T cell.
